# EUROPEAN PATENT APPLICATION

(11) **EP 1 743 639 A1**
(43) Date of publication of application: **17.01.2007**
(21) Application number: 05384023.7
(22) Date of filing: 15.07.2005
(51) Int. Cl.: A61K 31/415, A61P 7/02

(54) **Use of substituted pyrazoline compounds for the treatment of coagulation related diseases**

(71) Applicant: LABORATORIOS DEL DR. ESTEVE, S.A., 08041 Barcelona (ES)
(72) Inventor: Buschmann, Helmut Heinrich, 08960 Sant Just Desvern (Barcelona) (ES); Torrens Jover, Antonio, 08221 Terrasa (Barcelona) (ES); Yenes Minguez, Susana, 08750 Molins de Rei, Barcelona (ES); Mas Prio, José, 08191-Rubi, Barcelona (ES); Quintana Ruiz, Jordi Ramón, 08391-Tiana, Barcelona (ES); Dordal Zueras, Alberto, 08016 Barcelona (ES); Cuberes-Altisen, Maria Rosa, San Cugat del Valles, Barcelona (ES)

(57) **Abstract**

The present invention relates to the use of substituted pyrazoline compounds for the treatment of coagulation related diseases in humans and animals.

## Description

The present invention relates to the use of substituted pyrazoline compounds for the treatment of coagulation related diseases in humans and animals.

Coagulation related diseases, including the prophylaxis against unwanted coagulation, is a widespread health problem especially in elderly people but also in people with high risk factors, ultimately having as focus the danger of suffering a stroke. It goes without saying that this as one of the major reasons for death worldwide has received much public attention, thus - given the little choices a potential patient here has - leading to a clear medical need.

Thus, it was an object of the present invention to provide compounds for use as active substances in medicaments, which are suitable for the treatment of coagulation related diseases.

Said object was achieved by using the substituted pyrazoline compounds of general formula I given below, their stereoisomers, corresponding salts and corresponding solvates thereof.

It seems that these compounds have a marked effect on the lipid parameters of the metabolic syndrome.

Thus, in one of its aspects the present invention relates to the use of a substituted pyrazoline compounds of general formula I 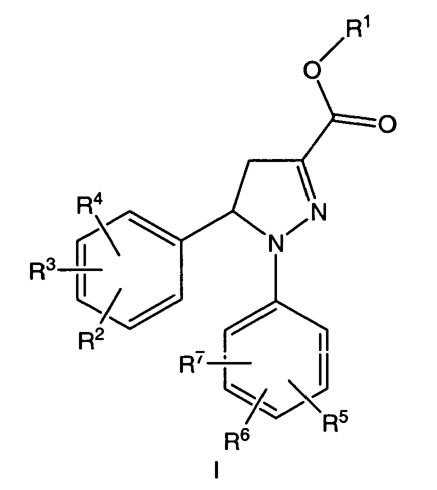 wherein
R¹ represents hydrogen or a linear or branched, substituted or unsubstituted, saturated or unsaturated, C₁₋₄-alkyl group,
R², R³ and R⁴ independently of each other represent hydrogen, a linear or branched C₁₋₆-alkyl group, a linear or branched C₁₋₆-alkoxy group, a halogen atom, CH₂F, CHF₂, CF₃, CN, OH, NO₂, -(C=O)-R⁸, SH, SR⁸, SOR⁸, SO₂R⁸, NH₂, NHR⁸, NR⁸R⁹, -(C=O)-NH₂, -(C=O)-NHR⁸ or -(C=O)-NR⁸R⁹ whereby R⁸ and R⁹ for each substituent independently represent linear or branched C₁₋₆ alkyl,
R⁵ and R⁶ independently of each other represent a linear or branched C₁₋₆₋alkyl group, a linear or branched C₁₋₆-alkoxy group, a halogen atom, CH₂F, CHF₂, CF₃, CN, OH, NO₂, -(C=O)-R¹⁰, SH, SR¹⁰, SOR¹⁰, NH₂, NHR¹⁰, NR¹⁰R¹¹, -(C=O)-NH₂, -(C=O)-NHR¹⁰ and -(C=O)-NR¹⁰R¹¹, whereby R¹⁰ and optionally R¹¹ for each substituent independently represent linear or branched C₁₋₆ alkyl;
R⁷ represents hydrogen, a linear or branched C₁₋₆-alkyl group, a linear or branched C₁₋₆-alkoxy group, a halogen atom, CH₂F, CHF₂, CF₃, CN, OH, NO₂, -(C=O)-R¹⁰, SH, SR¹⁰, SOR¹⁰, NH₂, NHR¹⁰, NR¹⁰R¹¹, -(C=O)-NH₂, -(C=O)-NHR¹⁰ and -(C=O)-NR¹⁰R¹¹, whereby R¹⁰ and optionally R¹¹ for each substituent independently represent linear or branched C₁₋₆ alkyl;
optionally in form of one of the stereoisomers, preferably enantiomers or diastereomers, a racemate or in form of a mixture of at least two of the stereoisomers, preferably enantiomers and/or diastereomers, in any mixing ratio, or a corresponding N-oxide thereof, or a corresponding salt thereof, or a corresponding solvate thereof,
for the manufacture of a medicament for the treatment of coagulation related diseases.

These compounds had a surprising effect on coagulation related diseases.

Coagulation related diseases does encompass as defined in this invention especially the risk of unwanted unphysiologically fast or high coagulation, or platelet aggregation. Thus it also includes the prophylactic or acute use of platelet aggregation inhibitors to ameliorate the perceived or treal threat coming with uncontrolled coagulation, stroke. As defined here, therefore, treatment does also include prophylaxis.

Preferred linear or branched, saturated or unsaturated aliphatic groups, which may be substituted by one or more substituents, may preferably be selected from the group consisting of methyl, ethyl, n-propyl, isopropyl, n-butyl, iso-butyl, sec-butyl, tert-butyl, n-pentyl, n-hexyl, n-heptyl, n-octyl, n-nonyl, n-decyl, vinyl, ethinyl, propenyl, propinyl, butenyl and butinyl.

In the context of this invention, alkyl and cycloalkyl radicals are understood as meaning saturated and unsaturated (but not aromatic), branched, unbranched and cyclic hydrocarbons, which can be unsubstituted or mono- or polysubstituted. In these radicals, C₁₋₂-alkyl represents C1- or C2-alkyl, C₁₋₃-alkyl represents C1-, C2- or C3-alkyl, C₁₋₄-alkyl represents C1-, C2-, C3- or C4-alkyl, C₁₋₅-alkyl represents C1-, C2-, C3-, C4-, or C5-alkyl, C₁₋₆-alkyl represents C1-, C2-, C3-, C4-, C5- or C6-alkyl, C₁₋₇-alkyl represents C1-, C2-, C3-, C4-, C5-, C6- or C7-alkyl, C₁₋₈-alkyl represents C1-, C2-, C3-, C4-, C5-, C6-, C7- or C8-alkyl, C₁₋₁₀-alkyl represents C1-, C2-, C3-, C4-, C5-, C6-, C7-, C8-, C9- or C10-alkyl and C₁₋₁₈-alkyl represents C1-, C2-, C3-, C4-, C5-, C6-, C7-, C8-, C9-, C10-, C11-, C12-, C13-, C14-, C15-, C16-, C17- or C18-alkyl. Furthermore, C₃₋₄-cycloalkyl represents C3- or C4-cycloalkyl, C₃₋₅₋cycloalkyl represents C3-, C4- or C5-cycloalkyl, C₃₋₆-cycloalkyl represents C3-, C4-, C5- or C6-cycloalkyl, C₃₋₇-cycloalkyl represents C3-, C4-, C5-, C6- or C7-cycloalkyl, C₃₋₈-cycloalkyl represents C3-, C4-, C5-, C6-, C7- or C8-cycloalkyl, C₄₋₅-cycloalkyl represents C4- or C5-cycloalkyl, C₄₋₆-cycloalkyl represents C4-, C5- or C6-cycloalkyl, C₄₋₇-cycloalkyl represents C4-, C5-, C6- or C7-cycloalkyl, C₅₋₆-cycloalkyl represents C5- or C6-cycloalkyl and C₅₋₇-cydoalkyl represents C5-, C6- or C7-cycloalkyl. In respect of cycloalkyl, the term also includes saturated cycloalkyls in which one or 2 carbon atoms are replaced by a heteroatom, S, N or O. However, mono- or polyunsaturated, preferably monounsaturated, cycloalkyls without a heteroatom in the ring also in particular fall under the term cycloalkyl as long as the cycloalkyl is not an aromatic system. The alkyl and cycloalkyl radicals are preferably methyl, ethyl, vinyl (ethenyl), propyl, allyl (2-propenyl), 1-propinyl, methylethyl, butyl, 1-methylpropyl, 2-methylpropyl, 1,1-dimethylethyl, pentyl, 1,1-dimethylpropyl, 1,2-dimethylpropyl, 2,2-dimethylpropyl, hexyl, 1-methylpentyl, cyclopropyl, 2-methylcyclopropyl, cyclopropylmethyl, cyclobutyl, cyclopentyl, cyclopentylmethyl, cyclohexyl, cycloheptyl, cyclooctyl, and also adamantyl, (if substituted also CHF₂, CF₃ or CH₂OH) as well as pyrazolinone, oxopyrazolinone, [1,4]-dioxane or dioxolane.

Here, in connection with alkyl and cycloalkyl - unless expressly defined otherwise - the term substituted in the context of this invention is understood as meaning replacement of at least one hydrogen radical by F, Cl, Br, I, NH₂, SH or OH, "polysubstituted" radicals being understood as meaning that the replacement takes effect both on different and on the same atoms several times with the same or different substituents, for example three times on the same C atom, as in the case of CF₃, or at different places, as in the case of -CH(OH)-CH=CH-CHCl₂. Particularly preferred substituents here are F, Cl and OH. In respect of cycloalkyl, the hydrogen radical can also be replaced by OC₁₋₃-alkyl or C₁₋₃-alkyl (in each case mono- or polysubstituted or unsubstituted), in particular methyl, ethyl, n-propyl, i-propyl, CF₃, methoxy or ethoxy.

The term (CH₂)₃₋₆ is to be understood as meaning -CH₂-CH₂-CH₂-, -CH₂-CH₂-CH₂₋CH₂-, -CH₂-CH₂-CH₂-CH₂-CH₂- and -CH₂-CH₂-CH₂-CH₂-CH₂-CH₂-, (CH₂)₁₋₄ is to be understood as meaning -CH₂-, -CH₂-CH₂-, -CH₂-CH₂-CH₂- and -CH₂-CH₂-CH₂-CH₂-, (CH₂)₄₋₅ is to be understood as meaning -CH₂-CH₂-CH₂-CH₂- and -CH₂-CH₂-CH₂₋CH₂-CH₂-, etc.

An aryl radical is understood as meaning ring systems with at least one aromatic ring but without heteroatoms even in only one of the rings. Examples are phenyl, naphthyl, fluoranthenyl, fluorenyl, tetralinyl or indanyl, in particular 9H-fluorenyl or anthracenyl radicals, which can be unsubstituted or monosubstituted or polysubstituted.

A heteroaryl radical is understood as meaning heterocyclic ring systems which have at least one unsaturated ring and can contain one or more heteroatoms from the group consisting of nitrogen, oxygen and/or sulfur and can also be mono- or polysubstituted. Examples which may be mentioned from the group of heteroaryls are furan, benzofuran, thiophene, benzothiophene, pyrrole, pyridine, pyrimidine, pyrazine, quinoline, isoquinoline, phthalazine, benzo-1,2,5-thiadiazole, benzothiazole, indole, benzotriazole, benzodioxolane, benzodioxane, carbazole and quinazoline.

Here, in connection with aryl and heteroaryl, substituted is understood as meaning substitution of the aryl or heteroaryl by R, OR, a halogen, preferably F and/or Cl, a CF₃, a CN, an NO₂, an NRR, a C₁₋₆-alkyl (saturated), a C₁₋₆₋alkoxy, a C₃₋₈₋cycloalkoxy, a C₃₋₈-cycloalkyl or a C₂₋₆-alkylene, with R meaning hydrogen or C₁₋₆₋alkyl.

The term "salt" is to be understood as meaning any form of the active compound used according to the invention in which it assumes an ionic form or is charged and is coupled with a counter-ion (a cation or anion) or is in solution. By this are also to be understood complexes of the active compound with other molecules and ions, in particular complexes which are complexed via ionic interactions. It especially includes physiologically acceptable salts, which is to be used equivalently to pharmacologically acceptable salts.

The term "physiologically acceptable salt" means in the context of this invention any salt that is physiologically tolerated (most of the time meaning not being toxic-especially not caused by the counter-ion) if used appropriately for a treatment especially if used on or applied to humans and/or mammals.

These physiologically acceptable salts can be formed with cations or bases and in the context of this invention is understood as meaning salts of at least one of the compounds used according to the invention - usually a (deprotonated) acid - as an anion with at least one, preferably inorganic, cation which is physiologically tolerated - especially if used on humans and/or mammals. The salts of the alkali metals and alkaline earth metals are particularly preferred, and also those with NH4, but in particular (mono)- or (di)sodium, (mono)- or (di)potassium, magnesium or calcium salts.

These physiologically acceptable salts can also be formed with anions or acids in the context of this invention is understood as meaning salts of at least one of the compounds used according to the invention - usually protonated, for example on the nitrogen - as the cation with at least one anion which are physiologically tolerated - especially if used on humans and/or mammals. By this is understood in particular, in the context of this invention, the salt formed with a physiologically tolerated acid, that is to say salts of the particular active compound with inorganic or organic acids which are physiologically tolerated - especially if used on humans and/or mammals. Examples of physiologically tolerated salts of particular acids are salts of:
hydrochloric acid, hydrobromic acid, sulfuric acid, methanesulfonic acid, formic acid, acetic acid, oxalic acid, succinic acid, malic acid, tartaric acid, mandelic acid, fumaric acid, lactic acid or citric acid.

The term "solvate" according to this invention is to be understood as meaning any form of the active compound according to the invention in which this compound has attached to it via non-covalent binding another molecule (most likely a polar solvent) especially including hydrates and alcoholates, e.g. methanolate.

Unless otherwise stated, the compounds of the invention are also meant to include compounds which differ only in the presence of one or more isotopically enriched atoms. For example, compounds having the present structures except for the replacement of a hydrogen by a deuterium or tritium, or the replacement of a carbon by ¹³C- or ¹⁴C-enriched carbon or ¹⁵N-enriched nitrogen are within the scope of this invention.

The invention also covers the use of any prodrug of the compounds described for the invention. Examples of well known methods of producing a prodrug of a given acting compound are known to those skilled in the art and can be found e.g. in Krogsgaard-Larsen et al., Textbook of Drugdesign and Discovery, Taylor & Francis (April 2002).

In a preferred embodiment of the invention the compound according to general formula I is selected from compounds according to general formula Ia or Ib or any mixture thereof

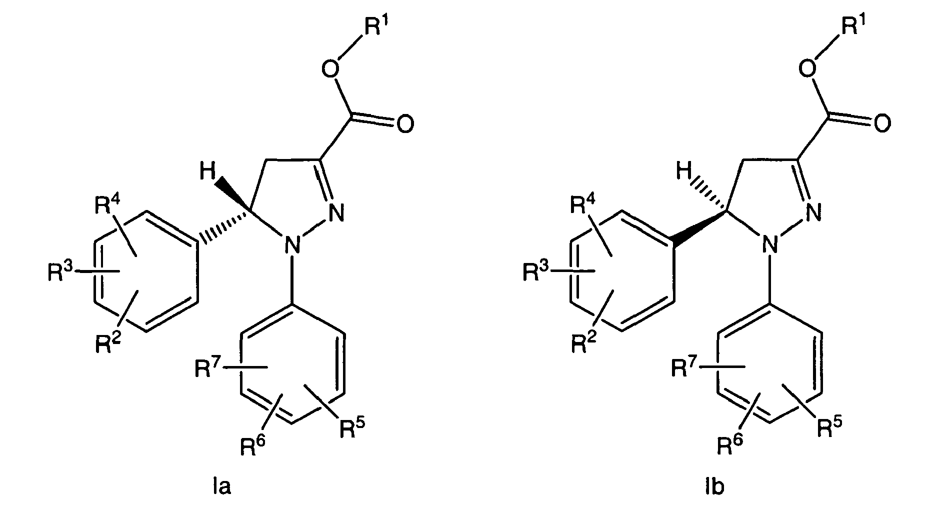

In a preferred embodiment of the invention for a compound according to formula I at least one of R², R³ or R⁴ represents hydrogen, while at least one of R², R³ or R⁴ is different from hydrogen.

In a preferred embodiment of the invention for a compound according to formula I R⁷ represents hydrogen.

In a preferred embodiment of the invention for a compound according to formula I R², R³ and R⁴ independently of each other represent hydrogen, a linear or branched C₁₋₆₋alkyl group, a halogen atom, or CF₃, preferably R², R³ and R⁴ independently of each other represent hydrogen, methyl, ethyl, F, Cl, Br and CF₃.

In a preferred embodiment of the invention for a compound according to formula I R⁵ and R⁶ independently of each other represent a linear or branched C₁₋₆-alkyl group, a halogen atom, or CF₃, preferably R⁵ and R⁶ independently of each other represent methyl, ethyl, F, Cl, Br and CF₃.

In a preferred embodiment of the invention for a compound according to formula I R² represents a chlorine atom in the 4-position of the phenyl ring, while R³ and R⁴ represent hydrogen.

In a preferred embodiment of the invention for a compound according to formula I R⁵ and R⁶ each represent a chlorine atoms in the 2- and 4-position of the phenyl ring, while R⁷ represents hydrogen.

In a preferred embodiment of the invention for a compound according to formula I R¹ represents hydrogen, methyl or ethyl, preferably hydrogen.

In a highly preferred further aspect of the invention the compound of general formula I is represented by a compound of general formula II 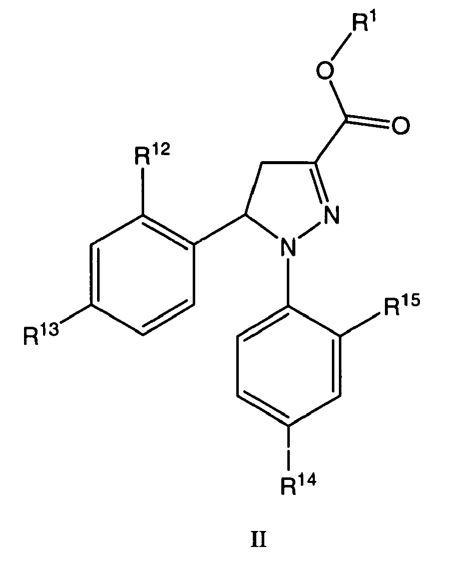 wherein
R¹ represents hydrogen or a linear or branched, substituted or unsubstituted, saturated or unsaturated, C₁₋₄-alkyl group,
R¹² or R¹³ independently of each other represent a linear or branched C₁₋₆₋alkyl group, a linear or branched C₁₋₆-alkoxy group, a halogen atom, CH₂F, CHF₂, CF₃, CN, OH, NO₂, SH, NH₂, hydrogen, methyl, ethyl, F, Cl, Br and CF₃,
R¹⁴ or R¹⁵ independently of each other represent a linear or branched C₁₋₆₋alkyl group, a linear or branched C₁₋₆-alkoxy group, a halogen atom, CH₂F, CHF₂, CF₃, CN, OH, NO₂, SH, NH₂, methyl, ethyl, F, Cl, Br and CF₃,
optionally in form of one of the stereoisomers, preferably enantiomers or diastereomers, a racemate or in form of a mixture of at least two of the stereoisomers, preferably enantiomers and/or diastereomers, in any mixing ratio, or a corresponding N-oxide thereof, or a corresponding salt thereof, or a corresponding solvate thereof.

In a preferred embodiment of the invention the compound according to general formula II is selected from compounds according to general formula IIa or IIb or any mixture thereof

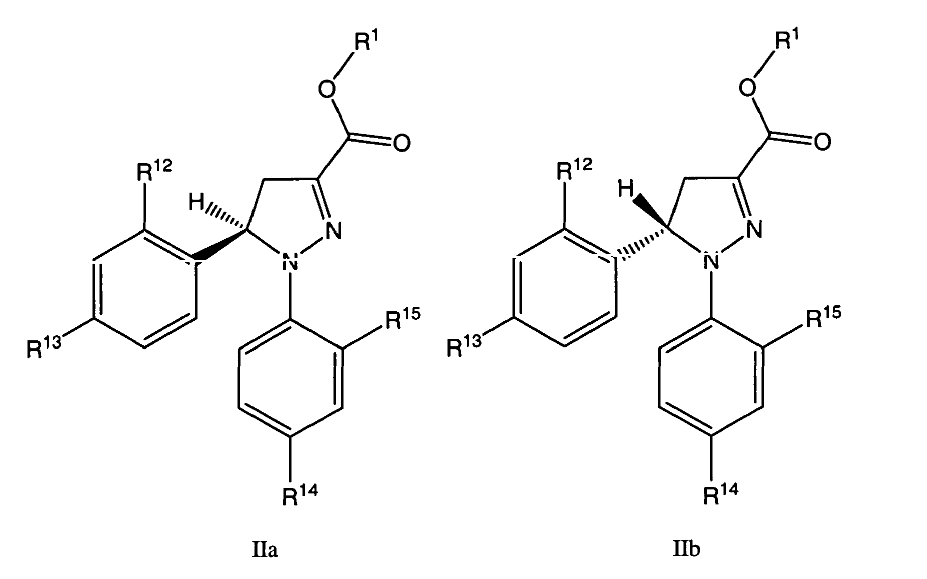

In a preferred embodiment of the invention for a compound according to formula II R¹² and R¹³ independently of each other represent hydrogen, a linear or branched C₁₋₆-alkyl group, a halogen atom, or CF₃, preferably R¹² and R¹³ independently of each other represent hydrogen, methyl, ethyl, F, Cl, Br and CF₃.

In a preferred embodiment of the invention for a compound according to formula II R¹⁴, and R¹⁵ independently of each other represent a linear or branched C₁₋₆-alkyl group, a halogen atom, or CF₃, preferably R¹⁴ and R¹⁵ independently of each other represent methyl, ethyl, F, Cl, Br and CF₃.

In a preferred embodiment of the invention for a compound according to formula II R¹³ represents Cl and R¹² represents hydrogen.

In a preferred embodiment of the invention for a compound according to formula II R¹⁴ and R¹⁵ each represent Cl.

In a preferred embodiment of the invention for a compound according to formula II R¹ represents hydrogen, methyl or ethyl, preferably hydrogen.

In another preferred embodiment the compound according to formula I or II is selected from the group consisting of:
- 5-(4-chloro-phenyl)-1-(2,4-dichlorophenyl)-4,5-dihydro-1H-pyrazol-3-carboxylic acid,
- (rac)-5-(4-chloro-phenyl)-1-(2,4-dichlorophenyl)-4,5-dihydro-1H-pyrazol-3-carboxylic acid,
- (R)-5-(4-chloro-phenyl)-1-(2,4-dichlorophenyl)-4,5-dihydro-1H-pyrazol-3-carboxylic acid,
- (S)-5-(4-chloro-phenyl)-1-(2,4-dichlorophenyl)-4,5-dihydro-1H-pyrazol-3-carboxylic acid,
or any mixture thereof
optionally in the form of a corresponding N-oxide, a corresponding salt or a corresponding solvate.

Another preferred aspect of the invention is the use of a substituted pyrazoline compound according to general formula X, 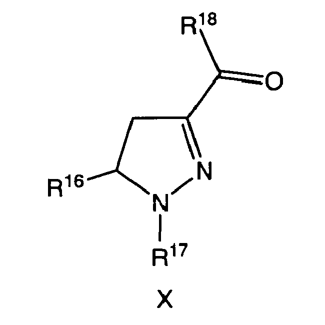 wherein
R¹⁶ represents an optionally at least mono-substituted phenyl group,
R¹⁷ represents an optionally at least mono-substituted phenyl group,
R¹⁸ represents a saturated or unsaturated, optionally at least mono-substituted, optionally at least one heteroatom as ring member containing cycloaliphatic group, which may be condensed with an optionally at least mono-substituted mono- or polycyclic ring system, or an optionally at least mono-substituted aryl or heteroaryl group, which may be condensed with an optionally at least mono-substituted mono- or polycyclic ring system, or an - NR¹⁹R²⁰-moiety,
R¹⁹ and R²⁰, identical or different, represent a hydrogen atom, an unbranched or branched, saturated or unsaturated, optionally at least mono-substituted aliphatic radical, a saturated or unsaturated, optionally at least mono-substituted, optionally at least one heteroatom as ring member containing cycloaliphatic group, which may be condensed with an optionally at least mono-substituted mono- or polycyclic ring system, or an optionally at least mono-substituted aryl or heteroaryl group, which may be condensed with an optionally at least mono-substituted mono- or polycyclic ring system and/or bonded via a linear or branched alkylene group, an ―SO₂-R²¹-moiety, or an ― NR²²R²³-moiety, with the proviso that R¹⁹ and R²⁰ do not identically represent hydrogen,
R²¹ represents a linear or branched, saturated or unsaturated, optionally at least mono-substituted aliphatic group, a saturated or unsaturated, optionally at least mono-substituted, optionally at least one heteroatom as ring member containing cycloaliphatic group, which may be condensed with a mono- or polycyclic ring-system, or an optionally at least mono-substituted aryl or heteroaryl group, which may be condensed with a mono- or polycyclic ring system and/or bonded via a linear or branched alkylene group,
R²² and R²³, identical or different, represent a hydrogen atom, an unbranched or branched, saturated or unsaturated, optionally at least mono-substituted aliphatic radical, a saturated or unsaturated, optionally at least mono-substituted, optionally at least one heteroatom as ring member containing cycloaliphatic group, which may be condensed with an optionally at least mono-substituted mono- or polycyclic ring system, or an optionally at least mono-substituted aryl or heteroaryl group, which may be condensed with an optionally at least mono-substituted mono- or polycyclic ring system and/or bonded via a linear or branched alkylene group,
optionally in form of one of the stereoisomers, preferably enantiomers or diastereomers, a racemate or in form of a mixture of at least two of the stereoisomers, preferably enantiomers and/or diastereomers, in any mixing ratio, or a corresponding N-oxide thereof, or a corresponding salt thereof, or a corresponding solvate thereof,
for the treatment of coagulation related diseases.

In a preferred embodiment of the invention in the compound according to general formula X R¹⁶ represents a phenyl group, which is optionally substituted by one or more substituents independently selected from the group consisting of a linear or branched C₁₋₆-alkyl group, a linear or branched C₁₋₆-alkoxy group, a halogen atom, CH₂F, CHF₂, CF₃, CN, OH, NO₂, -(C=O)-R', SH, SR', SOR', SO₂R', NH₂, NHR', NR'R", -(C=O)-NH₂, -(C=O)-NHR' and -(C=O)-NR'R" whereby R' and R" for each substituent independently represent linear or branched C₁₋₆ alkyl, preferably R¹⁶ represents a phenyl group, which is optionally substituted by one or more substituents selected from the group consisting of methyl, ethyl, F, Cl, Br and CF₃, more preferably R¹⁶ represents a phenyl group, which is mono-substituted with a chlorine atom in the 4-position.

In a preferred embodiment of the invention in the compound according to general formula X R¹⁷ represents a phenyl group, which is optionally substituted by one or more substituents independently selected from the group consisting of a linear or branched C₁₋₆-alkyl group, a linear or branched C₁₋₆-alkoxy group, a halogen atom, CH₂F, CHF₂, CF₃, CN, OH, NO₂, -(C=O)-R', SH, SR', SOR', SO₂R', NH₂, NHR', NR'R", -(C=O)-NH₂, -(C=O)-NHR' and -(C=O)-NR'R", whereby R' and optionally R" for each substituent independently represent linear or branched C₁₋₆ alkyl, preferably R¹⁷ represents a phenyl group, which is optionally substituted by one or more substituents independently selected from the group consisting of methyl, ethyl, F, Cl, Br and CF₃, more preferably R¹⁷ represents a phenyl group, which is di-substituted with two chlorine atoms in its 2- and 4-position.

In a preferred embodiment of the invention in the compound according to general formula X R¹⁸ represents a saturated or unsaturated, optionally at least mono-substituted, optionally at least one heteroatom as ring member containing C₃₋₈ cycloaliphatic group, which may be condensed with an optionally at least mono-substituted mono- or polycyclic ring system, or an optionally at least mono-substituted, 5- or 6-membered aryl or heteroaryl group, which may be condensed with an optionally at least mono-substituted mono- or polycyclic ring system, or an ― NR¹⁹R²⁰-moiety, preferably R¹⁸ represents a saturated, optionally at least mono-substituted, optionally one or more nitrogen-atoms as ring member containing C₃₋₈ cycloaliphatic group, which may be condensed with an optionally at least mono-substituted mono- or polycyclic ring system, or an -NR¹⁹R²⁰-moiety, more preferably R¹⁸ represents a pyrrolidinyl group, a piperidinyl group or a piperazinyl group, whereby each of these groups may be substituted with one or more C₁₋₆-alkyl groups, or an -NR¹⁸R¹⁹-moiety.

In a preferred embodiment of the invention in the compound according to general formula X R¹⁹ and R²⁰, identical or different, represent a hydrogen atom, an unbranched or branched, saturated or unsaturated, optionally at least mono-substituted C₁₋₆-aliphatic radical, a saturated or unsaturated, optionally at least mono-substituted, optionally at least one heteroatom as ring member containing C₃₋₈₋cycloaliphatic group, which may be condensed with an optionally at least mono-substituted mono- or polycyclic ring system, or an optionally at least mono-substituted, 5- or 6-membered aryl or heteroaryl group, which may be condensed with an optionally at least mono-substituted mono- or polycyclic ring system and/or bonded via a methylene (-CH₂-) or ethylene (-CH₂-CH₂)-group, an ―SO₂-R²¹-moiety, or an ―NR²²R²³-moiety, preferably one of these residues R¹⁹ and R²⁰ represents a hydrogen atom and the other one of these residues R¹⁹ and R²⁰ represents a saturated or unsaturated, optionally at least mono-substituted, optionally at least one heteroatom as ring member containing C₃₋₈-cycloaliphatic group, which may be condensed with an optionally at least mono-substituted mono- or polycyclic ring system, or an optionally at least mono-substituted, 5- or 6-membered aryl or heteroaryl group, which may be condensed with an optionally at least mono-substituted mono- or polycyclic ring system, an ―SO₂-R²¹-moiety, or an ―NR²²R²³⁻moiety, or R¹⁹ and R²⁰, identical or different, each represent a C₁₋₆ alkyl group, more preferably one of these residues R¹⁹ and R²⁰ represents a hydrogen atom and the other one of these residues R¹⁹ and R²⁰ represents an optionally at least mono-substituted pyrrolidinyl group, an optionally at least mono-substituted piperidinyl group, an optionally at least mono-substituted piperazinyl group, an optionally at least mono-substituted triazolyl group, an ―SO₂-R²¹-moiety, or an ―NR²²R²³-moiety, or R¹⁹ and R²⁰, identical or different, represent a methyl group, an ethyl group, an n-propyl group, an isopropyl group, an n-butyl group, a sec-butyl group or a tert.-butyl group.

In a preferred embodiment of the invention in the compound according to general formula X R²¹ represents a linear or branched, saturated or unsaturated, optionally at least mono-substituted C₁₋₆ aliphatic group, a saturated or unsaturated, optionally at least mono-substituted, optionally at least one heteroatom as ring member containing C₃₋₈ cycloaliphatic group, which may be condensed with a mono- or polycyclic ring-system, or an optionally at least mono-substituted, 5- or 6-membered aryl or heteroaryl group, which may be condensed with a mono- or polycyclic ring system and/or bonded via a methylene (-CH₂-) or ethylene (-CH₂-CH₂)-group, preferably R²¹ represents a C₁₋₆-alkyl group, a saturated, optionally at least mono-substituted cycloaliphatic group, which may be condensed with a mono- or polycyclic ring-system, or a phenyl group, which is optionally substituted with one or more C₁₋₆ alkyl groups.

In a preferred embodiment of the invention in the compound according to general formula X R²² and R²³, identical or different, represent a hydrogen atom, an unbranched or branched, saturated or unsaturated, optionally at least mono-substituted C₁₋₆ aliphatic radical, a saturated or unsaturated, optionally at least mono-substituted, optionally at least one heteroatom as ring member containing C₃₋₈ cycloaliphatic group, which may be condensed with an optionally at least mono-substituted mono- or polycyclic ring system, or an optionally at least mono-substituted, 5- or 6 membered aryl or heteroaryl group, which may be condensed with an optionally at least mono-substituted mono- or polycyclic ring system and/or bonded via a methylene (-CH₂-) or ethylene (-CH₂-CH₂)-group, preferably R²² and R²³, identical or different, represent a hydrogen atom or a C₁₋₆ alkyl radical.

In a preferred embodiment of the invention the compound according to general formula X is represented by a structure wherein
R¹⁶ represents a phenyl ring, which is mono-substituted with a halogen atom, preferably a chlorine atom, in its 4-position,
R¹⁷ represents a phenyl ring, which is di-substituted with two halogen atoms, preferably chlorine atoms, in its 2- and 4-position,
R¹⁸ represents a pyrrolidinyl group, a piperidinyl group, a piperazinyl group, a homo-piperazinyl group, a morpholinyl group, or an -NR¹⁹R²⁰-moiety,
R¹⁹ represents a hydrogen atom or a linear or branched C₁₋₆-alkyl group,
R²⁰ represents a linear or branched C₁₋₆ alkyl group, an -SO₂-R²¹-moiety, a pyrrolidinyl group, a piperidinyl group, a piperazinyl group, a homo-piperazinyl group, a morpholinyl group, a triazolyl group, whereby each of the heterocyclic rings may be substituted with one or more, identical or different, C₁₋₆-alkyl groups, and
R²¹ represents a phenyl group, which is optionally substituted with one or more C₁₋₆ alkyl groups, which may be identical or different,
optionally in form of one of the stereoisomers, preferably enantiomers or diastereomers, a racemate or in form of a mixture of at least two of the stereoisomers, preferably enantiomers and/or diastereomers, in any mixing ratio, or a corresponding N-oxide thereof, or a corresponding salt thereof, or a corresponding solvate thereof.

In a preferred embodiment of the invention at least one compound according to formula X is selected from the group consisting of:
N-piperidinyl-5-(4-chloro-phenyl)-1-(2,4-dichlorophenyl)-4,5-dihydro-1H-pyrazol-3-carboxamide,
(rac)-N-piperidinyl-5-(4-chloro-phenyl)-1-(2,4-dichlorophenyl)-4,5-dihydro-1H-pyrazol-3-carboxamide,
(R)-N-piperidinyl-5-(4-chloro-phenyl)-1-(2,4-dichlorophenyl)-4,5-dihydro-1H-pyrazol-3-carboxamide,
(S)-N-piperidinyl-5-(4-chloro-phenyl)-1-(2,4-dichlorophenyl)-4,5-dihydro-1H-pyrazol-3-carboxamide,
5-(4-Chloro-phenyl)-1-(2,4-dichloro-phenyl)-4,5-dihydro-1H-pyrazole-3-carboxylic acid-[1,2,4]-triazole-4-yl-amide,
5-(4-Chloro-phenyl)-1-(2,4-dichloro-phenyl)-4,5-dihydro-1H-pyrazole-3-carboxylic acid-(4-methyl-piperazin-1-yl)-amide,
5-(4-Chloro-phenyl)-1-(2,4-dichloro-phenyl)-4,5-dihydro-1H-pyrazole-3-carboxylic acid diethylamide,
[5-(4-Chloro-phenyl)-1-(2,4-dichloro-phenyl)-4,5-dihydro-1H-pyrazole-3-yl]-piperidine-1-yl-methanone,
N-[5-(4-Chloro-phenyl)-1-(2,4-dichlorophenyl)-4,5-dihydro-1H-pyrazole-3-carbonyl]-4-methylphenylsulfonamide,
optionally in the form of a corresponding N-oxide, a corresponding salt or a corresponding solvate.

Another preferred aspect of the invention is the use of a combination (A) of at least one substituted pyrazoline compound according to general formula X ― as described above - and at least one substituted pyrazoline compound according to general formula I or II ― as described above - for the manufacture of a medicament for the treatment of coagulation related diseases.

In a preferred embodiment of the use of the combination (A) according to the invention at least the following compounds are used:
- 5-(4-chloro-phenyl)-1-(2,4-dichlorophenyl)-4,5-dihydro-1H-pyrazol-3-carboxylic acid,
- (rac)-5-(4-chloro-phenyl)-1-(2,4-dichlorophenyl)-4,5-dihydro-1H-pyrazol-3-carboxylic acid,
- (R)-5-(4-chloro-phenyl)-1-(2,4-dichlorophenyl)-4,5-dihydro-1H-pyrazol-3-carboxylic acid, or
- (S)-5-(4-chloro-phenyl)-1-(2,4-dichlorophenyl)-4,5-dihydro-1H-pyrazol-3-carboxylic acid,
or any mixture thereof
and
- N-piperidinyl-5-(4-chloro-phenyl)-1-(2,4-dichlorophenyl)-4,5-dihydro-1H-pyrazol-3-carboxamide;
- (rac)-N-piperidinyl-5-(4-chloro-phenyl)-1-(2,4-dichlorophenyl)-4,5-dihydro-1H-pyrazol-3-carboxamide;
- (R)-N-piperidinyl-5-(4-chloro-phenyl)-1-(2,4-dichlorophenyl)-4,5-dihydro-1 H-pyrazol-3-carboxamide;
- (S)-N-piperidinyl-5-(4-chloro-phenyl)-1-(2,4-dichlorophenyl)-4,5-dihydro-1 H-pyrazol-3-carboxamide;
or any mixture thereof
each optionally in the form of a corresponding N-oxide, a corresponding salt or a corresponding solvate.

The process to acquire compounds according to general formula I and consequently also of general formula X is illustrated in scheme I given below:

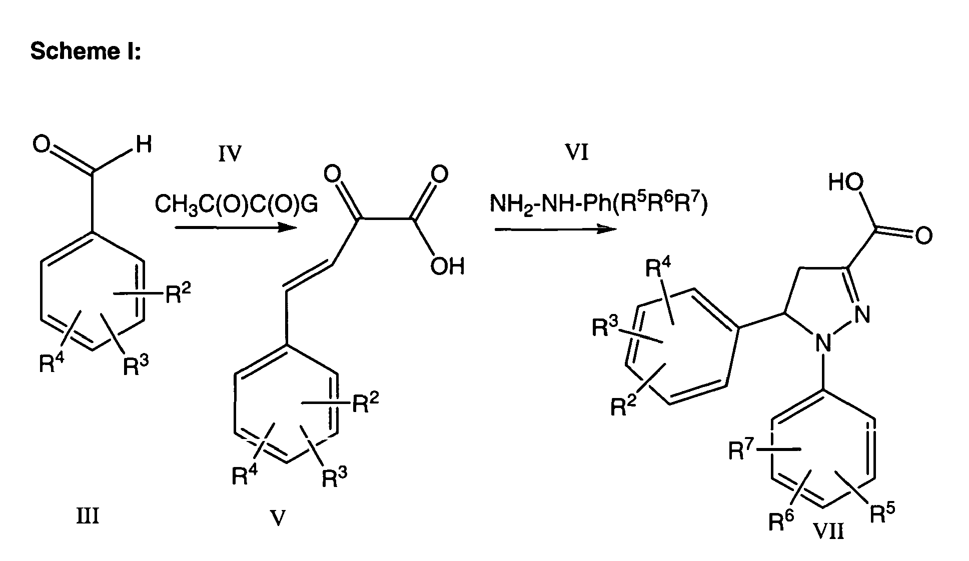

The reaction of the benzaldehyde compound of general formula III with a pyruvate compound of general formula IV is preferably carried out in the presence of at least one base, more preferably in the presence of an alkali metal hydroxide such as sodium hydroxide or potassium hydroxide or an alkali metal methoxide such as sodium methoxide, as described, for example, in Synthetic communications, 26(11), 2229-33, (1996). The respective description is hereby incorporated by reference and forms part of the disclosure. Preferably said reaction is carried out in a protic reaction medium such as a C₁₋₄ alkyl alcohol or mixtures of these.

Reaction temperature as well as the duration of the reaction may vary over a broad range. Preferred reaction temperatures range from -10 °C to the boiling point of the reaction medium. Suitable reaction times may vary for example from several minutes to several hours.

The reaction of the benzaldehyde compound of general formula III with a pyruvate compound of general formula IV is preferably carried out under acid catalysed conditions, more preferably by refluxing the mixture in dichloromethane in the presence of copper(II)trifluoromethanesulfonate as described, for example, in Synlett, (1), 147-149, 2001. The respective description is hereby incorporated by reference and forms part of the disclosure.

The reaction of the compound of general formula (V) with an optionally substituted phenyl hydrazin of general formula (VI) is preferably carried out in a suitable reaction medium such as C₁₋₄-alcohols or ethers such as dioxane or tetrahydrofurane or mixtures of at least two of these afore mentioned compounds. Also preferably, said reaction may be carried out in the presence of an acid, whereby the acid may be organic such as acetic acid and/or inorganic such as hydrochloric acid. Furthermore, the reaction may also be carried out in the presence of a base such as piperidine, piperazine, sodium hydroxide, potassium hydroxide, sodium methoxide or sodium ethoxide, or a mixture of at least two of these bases may also be used.

Reaction temperature as well as the duration of the reaction may vary over a broad range. Suitable reaction temperatures range from room temperature, i.e. approximately 25 °C to the boiling point of the reaction medium. Suitable reaction times may vary for example from several minutes to several hours.

The carboxylic group of the compound of general formula (VII) may be activated for further reactions by the introduction of a suitable leaving group according to conventional methods well known to those skilled in the art, leading to a compound according to general formula (Vila).

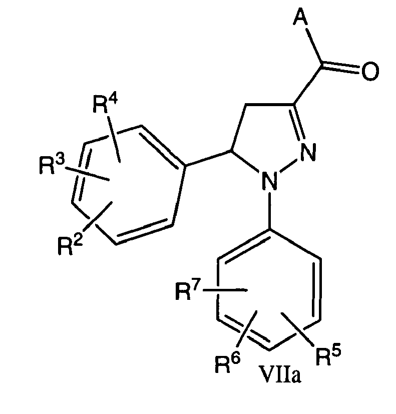

Preferably the compounds of general formula (VII) are transferred into an acid chloride, an acid anhydride, a mixed anhydride, a C₁₋₄ alkyl ester, an activated ester such as p-nitrophenylester. Other well known methods for the activation of acids include the activation with N,N-dicyclohexylcarbodiimide or benzotriazol-N-oxotris(dimethylamino) phosphonium hexafluorophosphate (BOP)).

If said activated compound of general formula (VIIa) is an acid chloride, it is preferably prepared by reaction of the corresponding acid of general formula (VII) with thionyl chloride or oxalyl chloride, whereby said chlorinating agent is also used as the solvent. Also preferably an additional solvent may be used. Suitable solvents include hydrocarbons such as benzene, toluene or xylene, halogenated hydrocarbons such as dichloromethane, chloroform or carbon tetrachloride, ethers such as diethyl ether, dioxane, tetrahydrofurane or dimethoxyethane. Mixtures of two or more solvents from one class or two or more solvents from different classes may also be used. Preferred reaction temperature range from 0º C to the boiling point of the solvent and reaction times from several minutes to several hours.

If said activated compound of general formula (VIIa) is a mixed anhydride, said anhydride may preferably be prepared, for example, by reaction of the corresponding acid of general formula (VIIa) with ethyl chloroformiate in the presence of a base such as triethylamine or pyridine, in a suitable solvent.

Following that the activated compound can be reacted with an alkyl-alcohol to arrive at compounds according to general formulas I or II with R¹ being a a linear or branched, substituted or unsubstituted, saturated or unsaturated, C₁₋₄-alkyl group.

In addition, as A represented in general formula VIIa represents a leaving group, via the reaction with an activating agent, said compound being optionally isolated and/or optionally purified, and at least one compound of general formula (VIIa) is reacted with a compound of general formula R¹⁸H, wherein R¹⁸ represents an -NR¹⁹R²⁰⁻moiety, wherein R¹⁹ and R²⁰ have the meaning given above for compounds of general formula X, to yield a substituted pyrazoline compound of general formula X, wherein R¹⁸ represents an -NR¹⁹R²⁰-moiety,

During the processes described above the protection of sensitive groups or of reagents may be necessary and/or desirable. The introduction of conventional protective groups as well as their removal may be performed by methods well-known to those skilled in the art.

If the substituted pyrazoline compounds of general formula I or II themselves are obtained in form of a mixture of stereoisomers, particularly enantiomers or diastereomers, said mixtures may be separated by standard procedures known to those skilled in the art, e.g. chromatographic methods or fractionalized crystallization with chiral reagents. It is also possible to obtain pure stereoisomers via stereoselective synthesis, especially using chiral bases like brucine, quinine, (-)-Cinchonidine, (+)-Cinchonine or R-(+)-1-Phenylethylamine.

In a process for the preparation of salts of substituted pyrazoline compounds of general formula I or II and stereoisomers thereof, at least one compound of general formula I or II having at least one basic group is reacted with at least one inorganic and/or organic acid, preferably in the presence of a suitable reaction medium. Suitable reaction media include, for example, any of the ones given above. Suitable inorganic acids include hydrochloric acid, hydrobromic acid, phosphoric acid, sulfuric acid, nitric acid, suitable organic acids are e.g. citric acid, maleic acid, fumaric acid, tartaric acid, or derivatives thereof, p-toluenesulfonic acid, methanesulfonic acid or camphersulfonic acid.

In a process for the preparation of salts of substituted pyrazoline compounds of general formula I or II or stereoisomers thereof, at least one compound of general formula I or II having at least one acidic group is reacted with one or more suitable bases, preferably in the presence of a suitable reaction medium. Suitable bases are e.g. hydroxides, carbonates or alkoxides, which include suitable cations, derived e.g. from alkaline metals, alkaline earth metals or organic cations, e.g. [NHₙR₄₋ₙ]⁺, wherein n is 0, 1, 2, 3 or 4 and R represents a branched or unbranched C₁₋₄-alkyl-radical. Suitable reaction media are, for example, any of the ones given above.

Solvates, preferably hydrates, of the substituted pyrazoline compounds of general formula I or II, of corresponding stereoisomers, of corresponding N-oxides or of corresponding salts thereof may also be obtained by standard procedures known to those skilled in the art.

Substituted pyrazoline compounds of general formula I or II, which comprise nitrogen-atom containing saturated, unsaturated or aromatic rings may also be obtained in the form of their N-oxides by methods well known to those skilled in the art.

The purification and isolation of the substituted pyrazoline compounds of general formula I or II, of a corresponding stereoisomer, or salt, or solvate or any intermediate thereof may, if required, be carried out by conventional methods known to those skilled in the art, e.g. chromatographic methods or recrystallization.

The substituted pyrazoline compounds of general formula I, la, Ib, II, Ila, IIb and X given below, their corresponding N-oxides, corresponding salts thereof and corresponding solvates are toxicologically acceptable and are therefore suitable as pharmaceutical active substances.

The medicament according to the present invention may be in any form suitable for the application to humans and/or animals, preferably humans including infants, children and adults and can be produced by standard procedures known to those skilled in the art. The composition of the medicament may vary depending on the route of administration.

The medicament of the present invention may for example be administered parentally in combination with conventional injectable liquid carriers, such as water or suitable alcohols. Conventional pharmaceutical excipients for injection, such as stabilizing agents, solubilizing agents, and buffers, may be included in such injectable compositions. These medicaments may for example be injected intramuscularly, intraperitoneally, or intravenously.

Solid oral compositions (which are preferred as are liquid ones) may be prepared by conventional methods of blending, filling or tabletting. Repeated blending operations may be used to distribute the active agent throughout those compositions employing large quantities of fillers. Such operations are conventional in the art. The tablets may for example be prepared by wet or dry granulation and optionally coated according to the methods well known in normal pharmaceutical practice., in particular with an enteric coating.

The mentioned formulations will be prepared using standard methods such as those described or referred to in the Spanish and US Pharmacopeias and similar reference texts.

Medicaments according to the present invention may also be formulated into orally administrable compositions containing one or more physiologically compatible carriers or excipients, in solid or liquid form. These compositions may contain conventional ingredients such as binding agents, fillers, lubricants, and acceptable wetting agents. The compositions may take any convenient form, such as tablets, pellets, capsules, lozenges, aqueous or oily solutions, suspensions, emulsions, or dry powdered forms suitable for reconstitution with water or other suitable liquid medium before use, for immediate or retarded release.

The liquid oral forms for administration may also contain certain additives such as sweeteners, flavoring, preservatives, and emulsifying agents. Non-aqueous liquid compositions for oral administration may also be formulated, containing edible oils. Such liquid compositions may be conveniently encapsulated in e.g., gelatin capsules in a unit dosage amount.

The compositions of the present invention may also be administered topically or via a suppository.

The daily dosage for humans and animals may vary depending on factors that have their basis in the respective species or other factors, such as age, sex, weight or degree of illness and so forth. The daily dosage for humans may preferably be in the range from1 to 2000, preferably 1 to 1500, more preferably 1 to 1000 milligrams of active substance to be administered during one or several intakes per day.

Another aspect of the invention is a combination (B) of at least one compound of either a substituted pyrazoline compound according to general formula X ― as described above ― or one substituted pyrazoline compound according to general formula I or II ― as described above ― with at least one antithrombotic agent.

Antithrombotics: Drugs that reduce blood coagulation (anticoagulants) or platelet activation (antiplatelet agents).

Anticoagulant: An agent that slows the blood clotting process. An anticoagulant is a substance that prevents coagulation; that is, it stops blood from clotting. A group of pharmaceuticals called anticoagulants can be used in vivo as a medication for thrombotic disorders. Some chemical compounds are used in medical equipment, such as test tubes, blood transfusion bags, and renal dialysis equipment.

Antiplatelet agent: A drug that prevents a type of cell in the blood called a platelet from clumping together. These clumps can often lead to blood clots that can block arteries, causing a heart attack or stroke.

For further details it can be referred to e.g. S.A. Mousa, Anticoagulants, Antiplatelets, and Thrombolytics (Methods in Molecular Medicine), Humana Press (November 1, 2003); Anticoagulants - A Medical Dictionary, Bibliography, and Annotated Research Guide to Internet References, Icon Health Publications (August 31, 2004); and K. Othmer, Blood, Coagulants and Anticoagulants to Cardiovascular Agents, Volume 4, Encyclopedia of Chemical Technology, 3rd Edition, Wiley-Interscience; (December 26, 1978).

A platelet aggregation inhibitor is defined as a compound being able to prolong - by inhibiting the ability of the platelets to aggregate - the coagulation time, as measurable in form of various tests known in the art especial specialists/doctors for internal medicine. Compounds which belong into that group include salicylates, especial acetyl salic acid, as well as coumarins.

In another preferred embodiment the antithrombotic agent may be selected from the following group:
**vitamin K antagonists,** such as dicoumarol, phenindion, warfarin, phenprocoumon, acenocoumarol, ethylbiscoumacetate, clorindion, diphenadion, and tioclomarol;
**heparin and its derivatives,** such as heparin, antithrombin III, dalteparin, enoxaparin, nadroparin, parnaparin, reviparin, danaparoid, tinzaparin, sulodexid, bemiparin, and certoparin;
**thrombocyte aggregation inhibitors** (except heparin and its derivatives), such as ditazol, cloricromen, picotamid, clopidogrel, ticlopidin, acetylsalicylic acid, dipyridamol, carbasalat calcium, epoprostenol, indobufen, iloprost, sulfinpyrazon, abciximab, anagrelid, aloxiprin, eptifibatid, tirofiban, triflusal, beraprost, and intrifiban;
**enzymes**, such as streptokinase, alteplase, anistreplase, urokinase, fibrinolysin, brinase, reteplase, saruplase, ancrod, drotecogin alfa (activated), tenecteplase, and protein C;
**direct thrombin inhibitors**, such as desirudin, lepirudin, argatroban, melagatran, und ximelagatran;
**further antithrombotic agents**, such as defibrotid, chondroitinsulfate B, fondaparinux, and sodium pentosanpolysulfate.

Suitable antithrombotic compounds are, for example, disclosed in US 6,905,688 according to which anticoagulants include, but are not limited to, heparin, low molecular weight heparin, warfarin sodium (e.g., COUMADIN®), dicumarol, 4-hydroxycoumarin, anisindione (e.g., MIRADON™), acenocoumarol (e.g., nicoumalone, SINTHROME™), indan-1,3-dione, phenprocoumon (e.g., MARCUMAR™), ethyl biscoumacetate (e.g., TROMEXAN™), and aspirin. Reference is also made to the Official German ATC-Index in the edition of 2005 (http://wido.de/fileadmin/wido/downloads/pdf_ arzneimitte)/wido_arz_atcddd_1204.pdf). The respective parts of the description are herewith incorporated by reference and form part of the disclosure.

Thus in a preferred embodiment of the combination (B) the platelet aggregation inhibitor is selected from salicylates, especial acetyl salic acid, and coumarins or their respective salts.

Thus in a preferred embodiment of the combination (B) the substituted pyrazoline compound according to general formula X, I or II respectively are selected from:.
- N-piperidinyl-5-(4-chloro-phenyl)-1-(2,4-dichlorophenyl)-4,5-dihydro-1H-pyrazol-3-carboxamide,
- (rac)-N-piperidinyl-5-(4-chloro-phenyl)-1-(2,4-dichlorophenyl)-4,5-dihydro-1H-pyrazol-3-carboxamide,
- (R)-N-piperidinyl-5-(4-chloro-phenyl)-1-(2,4-dichlorophenyl)-4,5-dihydro-1H-pyrazol-3-carboxamide,
- (S)-N-piperidinyl-5-(4-chloro-phenyl)-1-(2,4-dichlorophenyl)-4,5-dihydro-1H-pyrazol-3-carboxamide,
- 5-(4-Chloro-phenyl)-1-(2,4-dichloro-phenyl)-4,5-dihydro-1H-pyrazole-3-carboxylic acid-[1,2,4]-triazole-4-yl-amide,
- 5-(4-Chloro-phenyl)-1-(2,4-dichloro-phenyl)-4,5-dihydro-1H-pyrazole-3-carboxylic acid-(4-methyl-piperazin-1-yl)-amide,
- 5-(4-Chloro-phenyl)-1-(2,4-dichloro-phenyl)-4,5-dihydro-1H-pyrazole-3-carboxylic acid diethylamide,
- [5-(4-Chloro-phenyl)-1-(2,4-dichloro-phenyl)-4,5-dihydro-1H-pyrazole-3-yl]-piperidine-1-yl-methanone,
- N-[5-(4-Chloro-phenyl)-1-(2,4-dichlorophenyl)-4,5-dihydro-1H-pyrazole-3-carbonyl]-4-methylphenylsulfonamide,
- 5-(4-chloro-phenyl)-1-(2,4-dichlorophenyl)-4,5-dihydro-1H-pyrazol-3-carboxylic acid,
- (rac)-5-(4-chloro-phenyl)-1-(2,4-dichlorophenyl)-4,5-dihydro-1H-pyrazol-3-carboxylic acid,
- (R)-5-(4-chloro-phenyl)-1-(2,4-dichlorophenyl)-4,5-dihydro-1H-pyrazol-3-carboxylic acid,
- (S)-5-(4-chloro-phenyl)-1-(2,4-dichlorophenyl)-4,5-dihydro-1H-pyrazol-3-carboxylic acid,
or any mixture thereof
optionally in the form of a corresponding N-oxide, a corresponding salt or a corresponding solvate. or any mixture thereof
each optionally in the form of a corresponding N-oxide, a corresponding salt or a corresponding solvate.

Another aspect of the invention is a medicament comprising the combination (B), and optionally one or more pharmaceutically acceptable excipients.

Another aspect of the invention is the use of the combination (B) for the manufacture of a medicament for the prophylaxis and/or treatment of metabolic syndrome, especially weight independent, cardiovascular diseases especially fighting cardiovascular risk factors, influencing the blood parameters, especially the lipid parameters, diabetes, especially type II, glucose intolerance and insulin resistance, bone disorders, preferably osteoporosis (e.g. osteoporosis associated with a genetic predisposition, sex hormone deficiency, or ageing), cancer-associated bone disease or Paget's disease of bone; schizophrenia, anxiety, depression, epilepsy, neurodegenerative disorders, cerebellar disorders, spinocerebellar disorders, cognitive disorders, cranial trauma, head trauma, stroke, panic attacks, peripheric neuropathy, inflammation, glaucoma, migraine, Morbus Parkinson, Morbus Huntington, Morbus Alzheimer, Raynaud's disease, tremblement disorders, compulsive disorders, senile dementia, thymic disorders, tardive dyskinesia, bipolar disorders, medicament-induced movement disorders, dystonia, endotoxemic shock, hemorragic shock, hypotension, insomnia, immunologic disorders, sclerotic plaques, vomiting, diarrhea, asthma, memory disorders, pruritus, pain, or for potentiation of the analgesic effect of narcotic and non-narcotic analgesics, or for influencing intestinal transit; alcohol abuse and/or alcohol addiction, nicotine abuse and/or nicotine addiction, drug abuse and/or drug addiction and/or medicament abuse and/or medicament addiction, preferably drug abuse and/or drug addiction and/or nicotine abuse and/or nicotine addiction; cancer, preferably for the prophylaxis and/or treatment of one or more types of cancer selected from the group consisting of brain cancer, bone cancer, lip cancer, mouth cancer, esophageal cancer, stomach cancer, liver cancer, bladder cancer, pancreas cancer, ovary cancer, cervical cancer, lung cancer, breast cancer, skin cancer, colon cancer, bowel cancer and prostate cancer, more preferably for the prophylaxis and/or treatment of one or more types of cancer selected from the group consisting of colon cancer, bowel cancer and prostate cancer; food intake disorders, preferably bulimia, anorexia, cachexia, obesity and/or type II diabetus mellitus (non-insuline dependent diabetes mellitus), more preferably obesity; psychosis; disorders of the central nervous system, disorders of the immune system, disorders of the cardiovascular system, disorders of the endocrinous system, disorders of the respiratory system, disorders of the gastrointestinal tract or reproductive disorders.

Another preferred aspect of the invention is also a method of treatment encompassing all the abovementioned uses, wherein the substituted pyrazoline hydrates according to the invention is applied to a person in need thereof, treating metabolic syndrome, especially weight independent, cardiovascular diseases especially fighting cardiovascular risk factors, influencing the blood parameters, especially the lipid parameters, diabetes, especially type II, glucose intolerance and insulin resistance, bone disorders, preferably osteoporosis (e.g. osteoporosis associated with a genetic predisposition, sex hormone deficiency, or ageing), cancer-associated bone disease or Paget's disease of bone; schizophrenia, anxiety, depression, epilepsy, neurodegenerative disorders, cerebellar disorders, spinocerebellar disorders, cognitive disorders, cranial trauma, head trauma, stroke, panic attacks, peripheric neuropathy, inflammation, glaucoma, migraine, Morbus Parkinson, Morbus Huntington, Morbus Alzheimer, Raynaud's disease, tremblement disorders, compulsive disorders, senile dementia, thymic disorders, tardive dyskinesia, bipolar disorders, medicament-induced movement disorders, dystonia, endotoxemic shock, hemorragic shock, hypotension, insomnia, immunologic disorders, sclerotic plaques, vomiting, diarrhea, asthma, memory disorders, pruritus, pain, or for potentiation of the analgesic effect of narcotic and non-narcotic analgesics, or for influencing intestinal transit; alcohol abuse and/or alcohol addiction, nicotine abuse and/or nicotine addiction, drug abuse and/or drug addiction and/or medicament abuse and/or medicament addiction, preferably drug abuse and/or drug addiction and/or nicotine abuse and/or nicotine addiction; cancer, preferably for the prophylaxis and/or treatment of one or more types of cancer selected from the group consisting of brain cancer, bone cancer, lip cancer, mouth cancer, esophageal cancer, stomach cancer, liver cancer, bladder cancer, pancreas cancer, ovary cancer, cervical cancer, lung cancer, breast cancer, skin cancer, colon cancer, bowel cancer and prostate cancer, more preferably for the prophylaxis and/or treatment of one or more types of cancer selected from the group consisting of colon cancer, bowel cancer and prostate cancer; food intake disorders, preferably bulimia, anorexia, cachexia, obesity and/or type II diabetus mellitus (non-insuline dependent diabetes mellitus), more preferably obesity; psychosis; disorders of the central nervous system, disorders of the immune system, disorders of the cardiovascular system, disorders of the endocrinous system, disorders of the respiratory system, disorders of the gastrointestinal tract or reproductive disorders.

Another preferred aspect of the invention is also a method of treatment encompassing all the abovementioned uses, wherein the substituted pyrazoline hydrates according to the invention is applied to a person in need thereof, treating coagulation related diseases.

The present invention is illustrated below with the aid of examples. These illustrations are given solely by way of example and are not intended to limit the present invention.

### Examples:

### Example 0 represent a compound according to formula I or II.

### Example 0:

### 5-(4-chlorophenyl)-1-(2,4-dichlorophenyl)-4,5-dihydro-pyrazole-3-carboxylic acid

### a) 4-(4-chlorophenyl)-2-oxo-3-butenoic acid

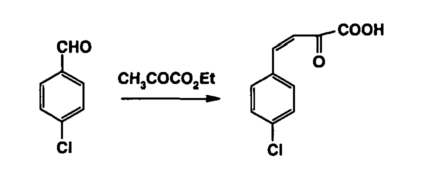

In a three neck flask p-chlorobenzaldehyde (13,3 g, 95 mmoles) and ethyl pyruvate (10 g, 86 mmoles) were dissolved in 150 ml of absolute ethanol.The solution was ice-cooled to 0°C and an aqueous solution of NaOH (3.8 g in 45 mL water) was added dropwise keeping the temperature below or equal to 10°C, whereby a yellow-orange colored precipitate was formed. The reaction mixture was stirred for 1 hour at 0°C and an additional 1.5 hours at room temperature (approximately 25 °C). Afterwards the reaction mixture was cooled down to approximately 5°C and the insoluble sodium salt of 4-(4-chlorophenyl)-2-oxo-3-butenoic acid was isolated by filtration.

The filtrate was left in the refrigerator overnight, whereby more precipitate is formed, which was filtered off, combined with the first fraction of the salt and washed with diethyl ether. The sodium salt of 4-(4-chlorophenyl)-2-oxo-3-butenoic acid was then treated with a solution of 2N HCl, stirred for some minutes and solid 4-(4-chlorophenyl)-2-oxo-3-butenoic acid was separated via filtration and dried to give 12.7 g of the desired product (70% of theoretical yield).

IR (KBr, cm⁻¹): 3500-2500, 1719,3, 1686,5, 1603,4, 1587,8, 1081,9.
¹H NMR(CDCl₃, δ): 7,4 (d, J=8,4Hz, 2H), 7,5 (d, J=16,1Hz, 1 H), 7,6 (d, J=8,4Hz, 2H), 8,1 (d, J=16,1 Hz, 1 H).

### a2) 5-(4-chlorophenyl)-1-(2,4-dichlorophenyl)-4,5-dihydro-pyrazole-3-carboxylic acid

In an alternative route instead of using ethylpyruvate the salt CH₃-C(O)-C(O)-O⁻ Na⁺ (sodiumpyruvate) was used, dissolved ethanolic water.

### b) 5-(4-chlorophenyl)-1-(2,4-dichlorophenyl)-4,5-dihydro-pyrazole-3-carboxylic acid

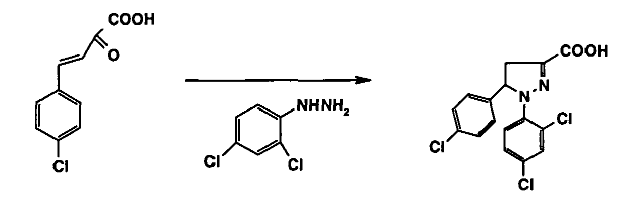

4-(4-chlorophenyl)-2-oxo-3-butenoic acid obtained according to step a) (12.6 g, 60 mmoles), 2,4-dichlorophenylhydrazine hydrochloride (12.8 g, 60 mmoles) and glacial acetic acid (200 mL) were mixed under a nitrogen atmosphere and heated to reflux for 4 hours, cooled down to room temperature (approximately 25 °C) and given into ice-water, whereby a sticky mass was obtained, which was extracted with methylene chloride. The combined methylene chloride fractions were washed with water, dried with sodium sulfate, filtered and evaporated to dryness to give a pale yellow solid (12.7 g, 57% of theoretical yield).

IR (KBr, cm⁻¹): 3200-2200, 1668,4, 1458, 1251,4, 1104,8.
¹H NMR (CDCl₃, δ): 3,3 (dd, 1 H), 3,7 (dd, 1 H), 5,9 (dd, 1 H), 7,09-7,25 (m, 7H).

The Examples 1 to 6 represent compounds according to formula X.

### Example 1:

### N-piperidinyl-5-(4-chlorophenyl)-1-(2,4-dichlorophenyl)-4,5-dihydro-1H-pyrazole-3-carboxamide

### (a) 5-(4-chlorophenyl)-1-(2,4-dichlorophenyl)-4,5-dihydro-pyrazole-3-carboxylic acid chloride

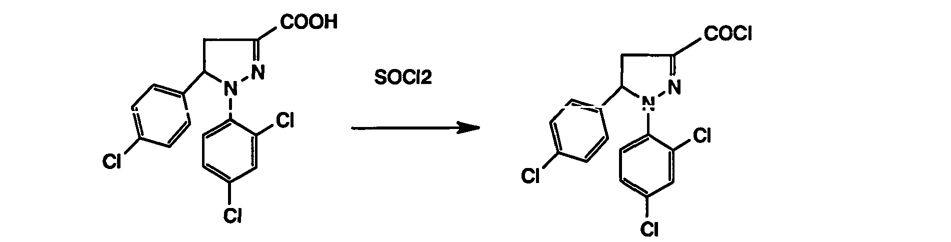

Under nitrogen atmosphere 5-(4-chlorophenyl)-1-(2,4-dichlorophenyl)-4,5-dihydro-pyrazole-3-carboxylic acid (2.5 g, 6.8 mmols) obtained according to Example 0 was dissolved in 4 mL of in thionyl chloride and heated to reflux for 2.5 hours. The excess thionyl chloride is removed from the reaction mixture under reduced pressure and the resulting crude residue (2.6 g) is used without any further purification.

IR (KBr, cm⁻¹): 1732,3, 1700, 1533,3, 1478,1, 1212,9, 826,6.

Starting from this compound compounds according to general formulas I and II wherein R¹ is a linear or branched C₁₋₄-alkyl group can be prepared reacting this compound with the appropriate alkyl alcohol.

### (b) N-piperidinyl-5-(4-chlorophenyl)-1-(2,4-dichlorophenyl)-4,5-dihydropyrazole-3-carboxamide

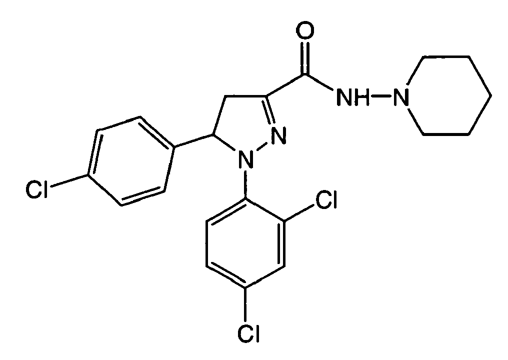

Under nitrogen atmosphere N-aminopiperidine (0.6 mL, 5.6 mmoles) and triethylamine (4 mL) were dissolved in methylene chloride (25 mL). The resulting mixture was ice-cooled down to 0°C and a solution of 5-(4-chlorophenyl)-1-(2,4-dichlorophenyl)-4,5-dihydro-pyrazole-3-carboxylic acid chloride obtained in step (b) in methylene chloride (15 mL) was added dropwise. The resulting reaction mixture was stirred at room temperature (approximately 25 °C) overnight. Afterwards the reaction mixture was washed with water, followed by a saturated aqueous solution of sodium bicarbonate, then again with water, dried over sodium sulfate, filtered and evaporated to dryness in a rotavapor. The resulting crude solid was crystallized from ethanol. The crystallized solid was removed via filtration and the mother liquors were concentrated to yield a second fraction of crystallized product. The two fractions were combined to give a total amount of 1.7 g (57% of theoretical yield) of N-piperidinyl-5-(4-chlorophenyl)-1-(2,4-dichlorophenyl)-4,5-dihydropyrazole-3-carboxamide having a melting point of 183-186°C.

IR (KBr, cm⁻¹): 3222,9, 2934,9, 1647,4, 1474,7, 1268,3, 815,6.
¹H NMR (CDCl₃, δ):1,4 (m, 2H), 1,7 (m, 4H), 2,8 (m, 4H), 3,3 (dd, J=6,1 y 18,3Hz, 1 H), 3,7 (dd, J=12,5 and 18,3 Hz, 1 H), 5,7 (dd, J=6,1 and 12,5 Hz, 1H), 7,0-7,2 (m, 6H), 7,4 (s, 1 H).

The compounds according to the following examples 2-6 have been prepared analogously to the process described in Example 1 in combination with Example 0.

### Example 2:

### 5-(4-Chloro-phenyl)-1-(2,4-dichloro-phenyl)-4,5-dihydro-1H-pyrazole-3-carboxylic acid-[1,2,4]triazol-4-yl amide

Melting point: 134-138 ºC.
IR (KBr, cm⁻¹): 3448, 1686, 1477, 1243, 1091, 821.
¹H NMR(CDCl₃, δ): 3,1 (dd, J=6,2 and 17,9Hz, 1H), 3,7 (dd, J=12,3 and
17,9Hz, 1 H), 5,9 (dd, J=6,2 and 12,3 Hz, 1 H), 7,2-7,5 (m, 7H), 8,7 (s, 2H), 12,0 (bs, 1H).

### Example 3:

### 5-(4-Chloro-phenyl)-1-(2,4-dichloro-phenyl)-4,5-dihydro-1H-pyrazole-3-carboxylic acid-(4-methyl-piperazin-1-yl)-amide hydrochloride

Melting point: 150-155ºC.
IR (KBr, cm⁻¹): 3433, 1685, 1477, 1296, 1246, 1088, 1014, 825.
¹H NMR (CDCl₃, δ): 2,7 (d, J=4,2Hz, 3H), 3,0-3,4 (m, 9H), 3,6 (dd, J=11,9 and 17,9 Hz, 1 H), 5,8 (dd, J=5,5 and 11,9 Hz, 1 H), 7,1 (d, J=8,4Hz, 2H), 7,25 (2d, J= 8,4 and 8,7 Hz, 3H), 7,4 (d, J=2,2Hz, 1 H), 7,5 (d, J=8,7Hz, 1H), 9,8 (s, 1 H). 11,2 (bs).

### Example 4:

### 5-(4-Chloro-phenyl)-1-(2,4-dichloro-phenyl)-4,5-dihydro-1H-pyrazole-3-carboxylic acid diethylamide

This compound was obtained in form of an oil.

IR (film, cm⁻¹): 2974, 1621, 1471, 1274, 1092, 820.
¹H NMR (CDCl₃, δ): 1,2 (m, 6H), 3,3-3,9 (m, 6H), 5,6 (dd, J=5,8 and 11,7 Hz, 1 H), 7-7,25 (m, 7H).

### Example 5:

### [5-(4-Chloro-phenyl)-1-(2,4-dichloro-phenyl)-4,5-dihydro-1H-pyrazol-3-yl]-piperidin-1-yl-methanone

Melting point: 105-110ºC.
IR (KBr, cm⁻¹): 2934, 1622, 1470, 1446, 1266, 1010, 817.
¹H NMR (CDCl₃, δ): 1,7 (m, 6H), 3,4 (dd, J=5,7 and 17,9Hz, 1 H), 3,7 (m, 3H), 3,9 (m, 2H), 5,6 (dd, J=6,1 y 11,9 Hz, 1 H), 7-7,25 (m, 7H).

### Example 6:

### N-[5-(4-Chloro-phenyl)-1-(2,4-dichloro-phenyl)-4,5-dihydro-1H-pyrazole-3-carbonyl]-4-methyl-phenylsulfonamide

This compound was obtained in form of an amorph solid.
IR (KBr, cm⁻¹): 1697, 1481, 1436, 1340, 1169, 1074, 853.
¹H NMR (CDCl₃, δ): 2,4 (s, 3H), 3,2 (dd, J=6,6 and 18,3Hz, 1 H), 3,6 (dd, J=12,8 and 18,3Hz, 1 H), 5,8 (dd, J=6,6 and 12,8Hz, 1 H), 7 (d, J=8,2Hz, 2H), 7,2 (s, 1 H), 7,3-7,4 (m, 6H), 8 (d, J=8,1 Hz, 2H), 9 (s, 1 H).

### Example 7:

### N-oxide of N-piperidinyl-5-(4-chlorophenyl)-1-(2,4-dichlorophenyl)-4,5-dihydropyrazole-3-carboxamide

Under nitrogen gas as an inert atmosphere N-piperidinyl-5-(4-chlorophenyl)-1-(2,4-dichlorophenyl)-4,5-dihydropyrazole-3-carboxamide (0,15 g, 332 mmoles) was dissolved in 7 ml of dichloromethane. The resulting solution was ice-cooled to 0 °C and m-chloroperbenzoic acid (0,204 g, 0,83 mmoles) added in several portions. After stirring for 15 minutes a control via thin layer chromatography showed that no starting material was remaining. A saturated solution of sodium bicarbonate was then slowly added, the organic phase separated, washed with water, dried over sodium sulfate and filtered. The filtered solution was evaporated to dryness and the crude product was purified via column chromatography yielding 78 mg (50 % of theoretical yield) of the N-oxide of N-piperidinyl-5-(4-chlorophenyl)-1-(2,4-dichlorophenyl)-4,5-dihydropyrazole-3-carboxamide in form of a white solid having a melting point of 115-120°C.

IR (KBr, cm⁻¹): 3202, 1678, 1654, 1474, 1309, 1107.
¹H-NMR (CDCl₃, δ): 1.6 (m, 2H), 1.8-2.0 (m, 4H), 2.55 (m, 2H), 3.3 (dd, J = 6.3 Hz and 18.2 Hz, 1 H), 3.7 (m, 3H), 5.8 (dd, J = 6.3 Hz and 12.5 Hz, 1 H), 7.0-7.3 (m, 7H), 8.5 (s, 1 H.)

## Claims

1. Use of at least one substituted pyrazoline compound of general formula I, 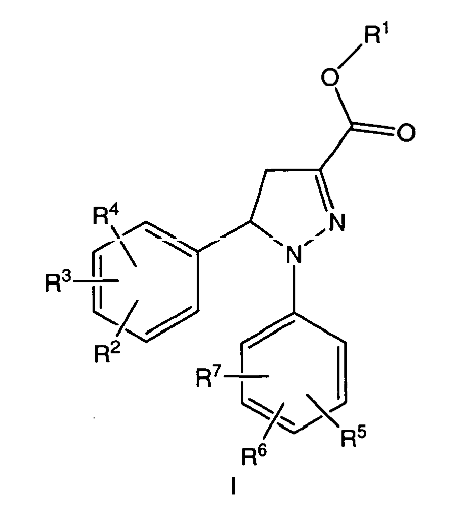 wherein
R¹ represents hydrogen or a linear or branched, substituted or unsubstituted, saturated or unsaturated, C₁₋₄-alkyl group,
R², R³ and R⁴ independently of each other represent hydrogen, a linear or branched C₁₋₆-alkyl group, a linear or branched C₁₋₆-alkoxy group, a halogen atom, CH₂F, CHF₂, CF₃, CN, OH, NO₂, -(C=O)-R⁸, SH, SR⁸, SOR⁸, SO₂R⁸, NH₂, NHR⁸, NR⁸R⁹, -(C=O)-NH₂, -(C=O)-NHR⁸ or -(C=O)-NR⁸R⁹ whereby R⁸ and R⁹ for each substituent independently represent linear or branched C₁₋₆ alkyl,
R⁵ and R⁶ independently of each other represent a linear or branched C₁₋₆₋alkyl group, a linear or branched C₁₋₆-alkoxy group, a halogen atom, CH₂F, CHF₂, CF₃, CN, OH, NO₂, -(C=O)-R¹⁰, SH, SR¹⁰, SOR¹⁰, NH₂, NHR¹⁰, NR¹⁰R¹¹, -(C=O)-NH₂, -(C=O)-NHR¹⁰ and -(C=O)-NR¹⁰R¹¹, whereby R¹⁰ and optionally R¹¹ for each substituent independently represent linear or branched C₁₋₆ alkyl;
R⁷ represents hydrogen, a linear or branched C₁₋₆-alkyl group, a linear or branched C₁₋₆-alkoxy group, a halogen atom, CH₂F, CHF₂, CF₃, CN, OH, NO₂, -(C=O)-R¹⁰, SH, SR¹⁰, SOR¹⁰, NH₂, NHR¹⁰, NR¹⁰R¹¹, -(C=O)-NH₂, -(C=O)-NHR¹⁰ and -(C=O)-NR¹⁰R¹¹, whereby R¹⁰ and optionally R¹¹ for each substituent independently represent linear or branched C₁₋₆ alkyl;
optionally in form of one of the stereoisomers, preferably enantiomers or diastereomers, a racemate or in form of a mixture of at least two of the stereoisomers, preferably enantiomers and/or diastereomers, in any mixing ratio, or a corresponding N-oxide thereof, or a corresponding salt thereof, or a corresponding solvate thereof,
for the manufacture of a medicament for the treatment of coagulation related diseases.

2. Use according to claim 1, **characterized in that** the compound according to general formula I is selected from compounds according to general formula la or Ib or any mixture thereof

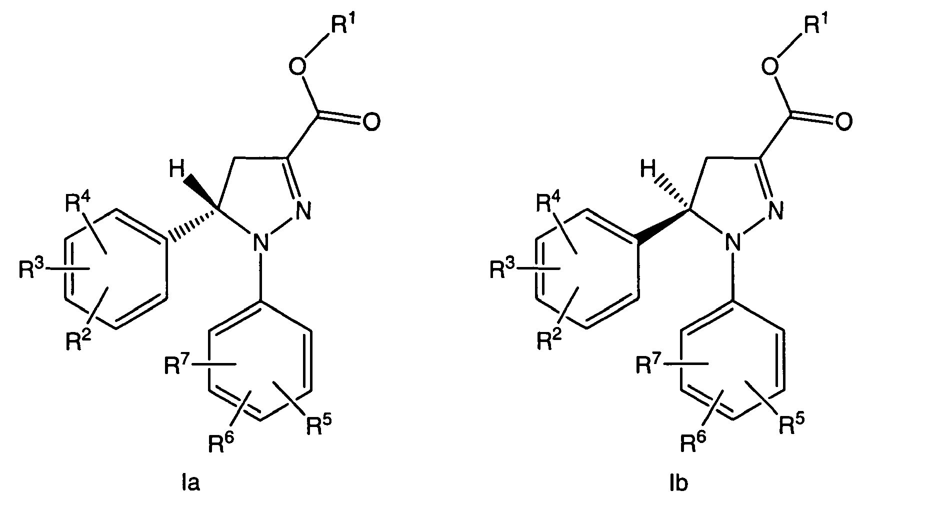

3. Use according to any of claims 1 or 2, **characterized in that** for the compound according to general formula I at least one of R², R³ or R⁴ represents hydrogen, while at least one of R², R³ or R⁴ is different from hydrogen.

4. Use according to any one of claims 1, 2 or 3, **characterized in that** for the compound according to general formula I R⁷ represents hydrogen.

5. Use according to any one of claims 1 to 4, **characterized in that** for the compound according to general formula I R², R³ and R⁴ independently of each other represent hydrogen, a linear or branched C₁₋₆-alkyl group, a halogen atom, or CF₃, preferably R², R³ and R⁴ independently of each other represent hydrogen, methyl, ethyl, F, Cl, Br and CF₃.

6. Use according to any one of claims 1 to 5, **characterized in that** for the compound according to general formula I R⁵ and R⁶ independently of each other represent a linear or branched C₁₋₆-alkyl group, a halogen atom, or CF₃, preferably R⁵ and R⁶ independently of each other represent methyl, ethyl, F, Cl, Br and CF₃.

7. Use according to any one of claims 1 to 6, **characterized in that** for the compound according to general formula I R² represents a chlorine atom in the 4-position of the phenyl ring, while R³ and R⁴ represent hydrogen.

8. Use according to any one of claims 1 to 7, **characterized in that** for the compound according to general formula I R⁵ and R⁶ each represent a chlorine atoms in the 2- and 4-position of the phenyl ring, while R⁷ represents hydrogen.

9. Use according to any one of claims 1 to 8, **characterized in that** for the compound according to general formula I R¹ represents hydrogen, methyl or ethyl, preferably hydrogen.

10. Use according to one or more of claims 1 to 9, **characterized in that** the compound according to general formula I is selected from a compound of general formula II 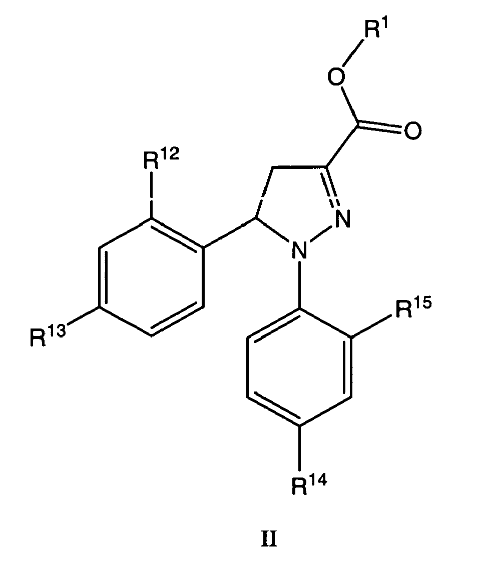 wherein
R¹ represents hydrogen or a linear or branched, substituted or unsubstituted, saturated or unsaturated, C₁₋₄-alkyl group,
R¹² or R¹³ independently of each other represent a linear or branched C₁₋₆₋alkyl group, a linear or branched C₁₋₆-alkoxy group, a halogen atom, CH₂F, CHF₂, CF₃, CN, OH, NO₂, SH, NH₂, hydrogen, methyl, ethyl, F, Cl, Br and CF₃,
R¹⁴ or R¹⁵ independently of each other represent a linear or branched C₁₋₆₋alkyl group, a linear or branched C₁₋₆-alkoxy group, a halogen atom, CH₂F, CHF₂, CF₃, CN, OH, NO₂, SH, NH₂, methyl, ethyl, F, Cl, Br and CF₃, optionally in form of one of the stereoisomers, preferably enantiomers or diastereomers, a racemate or in form of a mixture of at least two of the stereoisomers, preferably enantiomers and/or diastereomers, in any mixing ratio, or a corresponding N-oxide thereof, or a corresponding salt thereof, or a corresponding solvate thereof.

11. Use according to claim 10, **characterized in that** the compound according to general formula II is selected from compounds according to general formula IIa or IIb or any mixture thereof

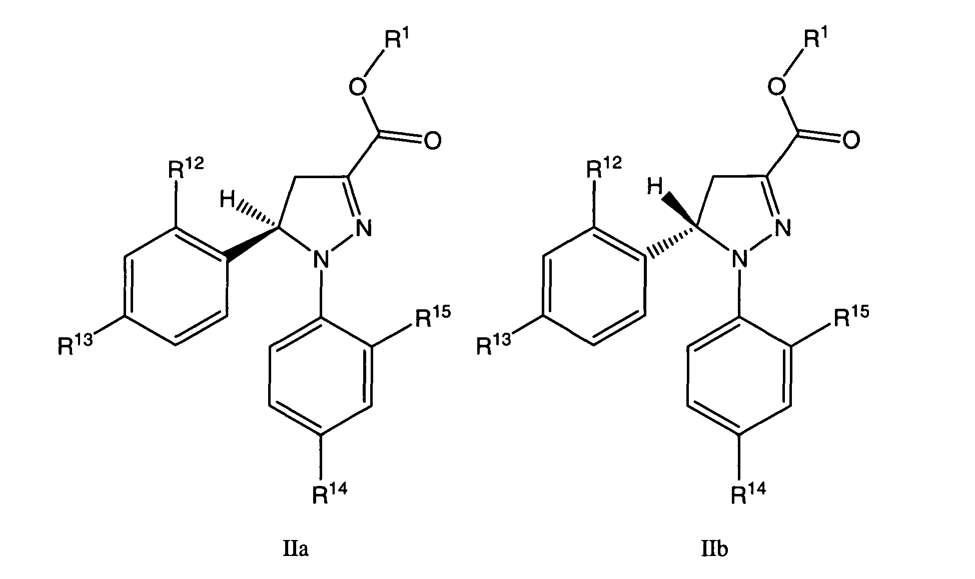

12. Use according to any of claims 10 or 11 **characterized in that** for the compound according to general formula II R¹² and R¹³ independently of each other represent hydrogen, a linear or branched C₁₋₆-alkyl group, a halogen atom, or CF₃, preferably R¹² and R¹³ independently of each other represent hydrogen, methyl, ethyl, F, Cl, Br and CF₃.

13. Use according to any one of claims 10, 11 or 12, **characterized in that** compound according to general formula II R¹⁴, and R¹⁵ independently of each other represent a linear or branched C₁₋₆-alkyl group, a halogen atom, or CF₃, preferably R¹⁴ and R¹⁵ independently of each other represent methyl, ethyl, F, Cl, Br and CF₃.

14. Use according to any one of claims 10 to 13, **characterized in that** compound according to general formula II R¹³ represents Cl and R¹² represents hydrogen.

15. Use according to any one of claims 10 to 14, **characterized in that** compound according to general formula II R¹⁴ and R¹⁵ each represent Cl.

16. Use according to any one of claims 10 to 15, **characterized in that** compound according to general formula II R¹ represents hydrogen, methyl or ethyl, preferably hydrogen.

17. Use according to one or more of claims 1 to 16, wherein the compound according to general formula II is selected from the group consisting of:
• 5-(4-chloro-phenyl)-1-(2,4-dichlorophenyl)-4,5-dihydro-1H-pyrazol-3-carboxylic acid,
• (rac)-5-(4-chloro-phenyl)-1-(2,4-dichlorophenyl)-4,5-dihydro-1H-pyrazol-3-carboxylic acid,
• (R)-5-(4-chloro-phenyl)-1-(2,4-dichlorophenyl)-4,5-dihydro-1H-pyrazol-3-carboxylic acid,
• (S)-5-(4-chloro-phenyl)-1-(2,4-dichlorophenyl)-4,5-dihydro-1H-pyrazol-3-carboxylic acid,
or any mixture thereof
optionally in the form of a corresponding N-oxide, a corresponding salt or a corresponding solvate.

18. Use of a substituted pyrazoline compound according to general formula X, 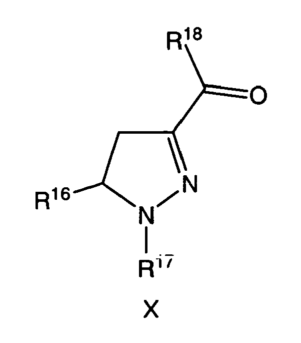 wherein
R¹⁶ represents an optionally at least mono-substituted phenyl group,
R¹⁷ represents an optionally at least mono-substituted phenyl group,
R¹⁸ represents a saturated or unsaturated, optionally at least mono-substituted, optionally at least one heteroatom as ring member containing cycloaliphatic group, which may be condensed with an optionally at least mono-substituted mono- or polycyclic ring system, or an optionally at least mono-substituted aryl or heteroaryl group, which may be condensed with an optionally at least mono-substituted mono- or polycyclic ring system, or an ― NR¹⁹R²⁰-moiety,
R¹⁹ and R²⁰, identical or different, represent a hydrogen atom, an unbranched or branched, saturated or unsaturated, optionally at least mono-substituted aliphatic radical, a saturated or unsaturated, optionally at least mono-substituted, optionally at least one heteroatom as ring member containing cycloaliphatic group, which may be condensed with an optionally at least mono-substituted mono- or polycyclic ring system, or an optionally at least mono-substituted aryl or heteroaryl group, which may be condensed with an optionally at least mono-substituted mono- or polycyclic ring system and/or bonded via a linear or branched alkylene group, an ―SO₂-R²¹-moiety, or an ― NR²²R²³-moiety, with the proviso that R¹⁹ and R²⁰ do not identically represent hydrogen,
R²¹ represents a linear or branched, saturated or unsaturated, optionally at least mono-substituted aliphatic group, a saturated or unsaturated, optionally at least mono-substituted, optionally at least one heteroatom as ring member containing cycloaliphatic group, which may be condensed with a mono- or polycyclic ring-system, or an optionally at least mono-substituted aryl or heteroaryl group, which may be condensed with a mono- or polycyclic ring system and/or bonded via a linear or branched alkylene group,
R²² and R²³, identical or different, represent a hydrogen atom, an unbranched or branched, saturated or unsaturated, optionally at least mono-substituted aliphatic radical, a saturated or unsaturated, optionally at least mono-substituted, optionally at least one heteroatom as ring member containing cycloaliphatic group, which may be condensed with an optionally at least mono-substituted mono- or polycyclic ring system, or an optionally at least mono-substituted aryl or heteroaryl group, which may be condensed with an optionally at least mono-substituted mono- or polycyclic ring system and/or bonded via a linear or branched alkylene group,
optionally in form of one of the stereoisomers, preferably enantiomers or diastereomers, a racemate or in form of a mixture of at least two of the stereoisomers, preferably enantiomers and/or diastereomers, in any mixing ratio, or a corresponding N-oxide thereof, or a corresponding salt thereof, or a corresponding solvate thereof,
for the manufacture of a medicament for the treatment of coagulation related dieases.

19. Use according to claim 18, **characterized in that** in the compound according to general formula X R¹⁶ represents a phenyl group, which is optionally substituted by one or more substituents independently selected from the group consisting of a linear or branched C₁₋₆-alkyl group, a linear or branched C₁₋₆-alkoxy group, a halogen atom, CH₂F, CHF₂, CF₃, CN, OH, NO₂, -(C=O)-R', SH, SR', SOR', SO₂R', NH₂, NHR', NR'R", -(C=O)-NH₂, -(C=O)-NHR' and -(C=O)-NR'R" whereby R' and R" for each substituent independently represent linear or branched C₁₋₆ alkyl, preferably R¹⁶ represents a phenyl group, which is optionally substituted by one or more substituents selected from the group consisting of methyl, ethyl, F, Cl, Br and CF₃, more preferably R¹⁶ represents a phenyl group, which is mono-substituted with a chlorine atom in the 4-position.

20. Use according to any of claims 18 or 19, **characterized in that** in the compound according to general formula X R¹⁷ represents a phenyl group, which is optionally substituted by one or more substituents independently selected from the group consisting of a linear or branched C₁₋₆-alkyl group, a linear or branched C₁₋₆-alkoxy group, a halogen atom, CH₂F, CHF₂, CF₃, CN, OH, NO₂, -(C=O)-R', SH, SR', SOR', SO₂R', NH₂, NHR', NR'R", -(C=O)-NH₂, -(C=O)-NHR' and -(C=O)-NR'R", whereby R' and optionally R" for each substituent independently represent linear or branched C₁₋₆ alkyl, preferably R¹⁷ represents a phenyl group, which is optionally substituted by one or more substituents independently selected from the group consisting of methyl, ethyl, F, Cl, Br and CF₃, more preferably R¹⁷ represents a phenyl group, which is di-substituted with two chlorine atoms in its 2- and 4-position.

21. Use according to any of claims 18 to 20, **characterized in that** in the compound according to general formula X R¹⁸ represents a saturated or unsaturated, optionally at least mono-substituted, optionally at least one heteroatom as ring member containing C₃₋₈ cycloaliphatic group, which may be condensed with an optionally at least mono-substituted mono- or polycyclic ring system, or an optionally at least mono-substituted, 5- or 6-membered aryl or heteroaryl group, which may be condensed with an optionally at least mono-substituted mono- or polycyclic ring system, or an -NR¹⁹R²⁰-moiety, preferably R¹⁸ represents a saturated, optionally at least mono-substituted, optionally one or more nitrogen-atoms as ring member containing C₃₋₈ cycloaliphatic group, which may be condensed with an optionally at least mono-substituted mono- or polycyclic ring system, or an -NR¹⁹R²⁰-moiety, more preferably R¹⁸ represents a pyrrolidinyl group, a piperidinyl group or a piperazinyl group, whereby each of these groups may be substituted with one or more C₁₋₆-alkyl groups, or an -NR¹⁸R¹⁹-moiety.

22. Use according to any of claims 18 to 21, **characterized in that** in the compound according to general formula X R¹⁹ and R²⁰, identical or different, represent a hydrogen atom, an unbranched or branched, saturated or unsaturated, optionally at least mono-substituted C₁₋₆-aliphatic radical, a saturated or unsaturated, optionally at least mono-substituted, optionally at least one heteroatom as ring member containing C₃₋₈-cycloaliphatic group, which may be condensed with an optionally at least mono-substituted mono- or polycyclic ring system, or an optionally at least mono-substituted, 5- or 6-membered aryl or heteroaryl group, which may be condensed with an optionally at least mono-substituted mono- or polycyclic ring system and/or bonded via a methylene (-CH₂-) or ethylene (-CH₂-CH₂)-group, an -SO₂-R²¹-moiety, or an -NR²²R²³-moiety, preferably one of these residues R¹⁹ and R²⁰ represents a hydrogen atom and the other one of these residues R¹⁹ and R²⁰ represents a saturated or unsaturated, optionally at least mono-substituted, optionally at least one heteroatom as ring member containing C₃₋₈-cycloaliphatic group, which may be condensed with an optionally at least mono-substituted mono- or polycyclic ring system, or an optionally at least mono-substituted, 5- or 6-membered aryl or heteroaryl group, which may be condensed with an optionally at least mono-substituted mono- or polycyclic ring system, an -SO₂-R²¹-moiety, or an -NR²²R²³⁻moiety, or R¹⁹ and R²⁰, identical or different, each represent a C₁₋₆ alkyl group, more preferably one of these residues R¹⁹ and R²⁰ represents a hydrogen atom and the other one of these residues R¹⁹ and R²⁰ represents an optionally at least mono-substituted pyrrolidinyl group, an optionally at least mono-substituted piperidinyl group, an optionally at least mono-substituted piperazinyl group, an optionally at least mono-substituted triazolyl group, an -SO₂-R²¹-moiety, or an -NR²²R²³-moiety, or R¹⁹ and R²⁰, identical or different, represent a methyl group, an ethyl group, an n-propyl group, an isopropyl group, an n-butyl group, a sec-butyl group or a tert.-butyl group.

23. Use according to any of claims 18 to 22, **characterized in that** in the compound according to general formula X R²¹ represents a linear or branched, saturated or unsaturated, optionally at least mono-substituted C₁₋₆ aliphatic group, a saturated or unsaturated, optionally at least mono-substituted, optionally at least one heteroatom as ring member containing C₃₋₈ cycloaliphatic group, which may be condensed with a mono- or polycyclic ring-system, or an optionally at least mono-substituted, 5- or 6-membered aryl or heteroaryl group, which may be condensed with a mono- or polycyclic ring system and/or bonded via a methylene (-CH₂-) or ethylene (-CH₂₋CH₂)-group, preferably R²¹ represents a C₁₋₆-alkyl group, a saturated, optionally at least mono-substituted cycloaliphatic group, which may be condensed with a mono-or polycyclic ring-system, or a phenyl group, which is optionally substituted with one or more C₁₋₆ alkyl groups.

24. Use according to any of claims 18 to 23, **characterized in that** in the compound according to general formula X R²² and R²³, identical or different, represent a hydrogen atom, an unbranched or branched, saturated or unsaturated, optionally at least mono-substituted C₁₋₆ aliphatic radical, a saturated or unsaturated, optionally at least mono-substituted, optionally at least one heteroatom as ring member containing C₃₋₈ cycloaliphatic group, which may be condensed with an optionally at least mono-substituted mono- or polycyclic ring system, or an optionally at least mono-substituted, 5- or 6 membered aryl or heteroaryl group, which may be condensed with an optionally at least mono-substituted mono- or polycyclic ring system and/or bonded via a methylene (-CH₂-) or ethylene (-CH₂-CH₂)-group, preferably R²² and R²³, identical or different, represent a hydrogen atom or a C₁₋₆ alkyl radical.

25. Use according to any of claims 18 to 24, **characterized in that** the compound according to general formula X is represented by a structure wherein
R¹⁶ represents a phenyl ring, which is mono-substituted with a halogen atom, preferably a chlorine atom, in its 4-position,
R¹⁷ represents a phenyl ring, which is di-substituted with two halogen atoms, preferably chlorine atoms, in its 2- and 4-position,
R¹⁸ represents a pyrrolidinyl group, a piperidinyl group, a piperazinyl group, a homo-piperazinyl group, a morpholinyl group, or an ―NR¹⁹R²⁰-moiety,
R¹⁹ represents a hydrogen atom or a linear or branched C₁₋₆-alkyl group,
R²⁰ represents a linear or branched C₁₋₆ alkyl group, an -SO₂-R²¹-moiety, a pyrrolidinyl group, a piperidinyl group, a piperazinyl group, a homo-piperazinyl group, a morpholinyl group, a triazolyl group, whereby each of the heterocyclic rings may be substituted with one or more, identical or different, C₁₋₆-alkyl groups, and
R²¹ represents a phenyl group, which is optionally substituted with one or more C₁₋₆ alkyl groups, which may be identical or different,
optionally in form of one of the stereoisomers, preferably enantiomers or diastereomers, a racemate or in form of a mixture of at least two of the stereoisomers, preferably enantiomers and/or diastereomers, in any mixing ratio, or a corresponding N-oxide thereof, or a corresponding salt thereof, or a corresponding solvate thereof.

26. Use according to any of claims 18 to 25, **characterized in that** at least one compound according to formula X is selected from the group consisting of:
N-piperidinyl-5-(4-chloro-phenyl)-1-(2,4-dichlorophenyl)-4,5-dihydro-1H-pyrazol-3-carboxamide,
(rac)-N-piperidinyl-5-(4-chloro-phenyl)-1-(2,4-dichlorophenyl)-4,5-dihydro-1H-pyrazol-3-carboxamide,
(R)-N-piperidinyl-5-(4-chloro-phenyl)-1-(2,4-dichlorophenyl)-4,5-dihydro-1H-pyrazol-3-carboxamide,
(S)-N-piperidinyl-5-(4-chloro-phenyl)-1-(2,4-dichlorophenyl)-4,5-dihydro-1H-pyrazol-3-carboxamide,
5-(4-Chloro-phenyl)-1-(2,4-dichloro-phenyl)-4,5-dihydro-1H-pyrazole-3-carboxylic acid-[1,2,4]-triazole-4-yl-amide,
5-(4-Chloro-phenyl)-1-(2,4-dichloro-phenyl)-4,5-dihydro-1H-pyrazole-3-carboxylic acid-(4-methyl-piperazin-1-yl)-amide,
5-(4-Chloro-phenyl)-1-(2,4-dichloro-phenyl)-4,5-dihydro-1H-pyrazole-3-carboxylic acid diethylamide,
[5-(4-Chloro-phenyl)-1-(2,4-dichloro-phenyl)-4,5-dihydro-1H-pyrazole-3-yl]-piperidine-1-yl-methanone,
N-[5-(4-Chloro-phenyl)-1-(2,4-dichlorophenyl)-4,5-dihvdro-1H-pyrazole-3-carbonyl]-4-methylphenylsulfonamide,
optionally in the form of a corresponding N-oxide, a corresponding salt or a corresponding solvate.

27. Use of a combination (A) comprising at least one substituted pyrazoline compound according to general formula X as defined in claims 18 to 26 and at least one substituted pyrazoline compound according to general formula I or II as defined in claims 1 to 17 for the manufacture of a medicament for the treatment of coagulation related diseases.

28. Use according to any of claim 27, **characterized in that** for the combination (A) at least the following compounds are used:
• 5-(4-chloro-phenyl)-1-(2,4-dichlorophenyl)-4,5-dihydro-1H-pyrazol-3-carboxylic acid,
• (rac)-5-(4-chloro-phenyl)-1-(2,4-dichlorophenyl)-4,5-dihydro-1H-pyrazol-3-carboxylic acid,
• (R)-5-(4-chloro-phenyl)-1-(2,4-dichlorophenyl)-4,5-dihydro-1H-pyrazol-3-carboxylic acid,
• (S)-5-(4-chloro-phenyl)-1-(2,4-dichlorophenyl)-4,5-dihydro-1H-pyrazol-3-carboxylic acid,
or any mixture thereof
and
• N-piperidinyl-5-(4-chloro-phenyl)-1-(2,4-dichlorophenyl)-4,5-dihydro-1H-pyrazol-3-carboxamide;
• (rac)-N-piperidinyl-5-(4-chloro-phenyl)-1-(2,4-dichlorophenyl)-4,5-dihydro-1 H-pyrazol-3-carboxamide;
• (R)-N-piperidinyl-5-(4-chloro-phenyl)-1-(2,4-dichlorophenyl)-4,5-dihydro-1 H-pyrazol-3-carboxamide; or
• (S)-N-piperidinyl-5-(4-chloro-phenyl)-1-(2,4-dichlorophenyl)-4,5-dihydro-1 H-pyrazol-3-carboxamide;
or any mixture thereof
each optionally in the form of a corresponding N-oxide, a corresponding salt or a corresponding solvate.

29. A combination (B) comprising at least one substituted pyrazoline compound according to general formula X as defined in claims 18 to 26 or at least one substituted pyrazoline compound according to general formula I or II as defined in claims 1 to 17 with at least one antithrombotic agent.

30. A combination (B) according to claim 30, **characterized in that** the antithrombotic agent is selected from the group consisting of
**vitamin K antagonists**, such as dicoumarol, phenindion, warfarin, phenprocoumon, acenocoumarol, ethylbiscoumacetate, clorindion, diphenadion, and tioclomarol;
**heparin and its derivatives,** such as heparin, antithrombin III, dalteparin, enoxaparin, nadroparin, parnaparin, reviparin, danaparoid, tinzaparin, sulodexid, bemiparin, and certoparin;
**thrombocyte aggregation inhibitors** (except heparin and its derivatives), such as ditazol, cloricromen, picotamid, clopidogrel, ticlopidin, acetylsalicylic acid, dipyridamol, carbasalat calcium, epoprostenol, indobufen, iloprost, sulfinpyrazon, abciximab, anagrelid, aloxiprin, eptifibatid, tirofiban, triflusal, beraprost, and intrifiban;
**enzymes**, such as streptokinase, alteplase, anistreplase, urokinase, fibrinolysin, brinase, reteplase, saruplase, ancrod, drotecogin alfa (activated), tenecteplase, and protein C;
**direct thrombin inhibitors**, such as desirudin, lepirudin, argatroban, melagatran, und ximelagatran;
**further antithrombotic agents**, such as defibrotid, chondroitinsulfate B, fondaparinux, and sodium pentosanpolysulfate.

31. A combination (B) according to claim 29 to 30, **characterized in that** at least one substituted pyrazoline compound according to general formulas X, I or II is selected from:
• N-piperidinyl-5-(4-chloro-phenyl)-1-(2,4-dichlorophenyl)-4,5-dihydro-1H-pyrazol-3-carboxamide,
• (rac)-N-piperidinyl-5-(4-chloro-phenyl)-1-(2,4-dichlorophenyl)-4,5-dihydro-1H-pyrazol-3-carboxamide,
• (R)-N-piperidinyl-5-(4-chloro-phenyl)-1-(2,4-dichlorophenyl)-4,5-dihydro-1H-pyrazol-3-carboxamide,
• (S)-N-piperidinyl-5-(4-chloro-phenyl)-1-(2,4-dichlorophenyl)-4,5-dihydro-1H-pyrazol-3-carboxamide,
• 5-(4-Chloro-phenyl)-1-(2,4-dichloro-phenyl)-4,5-dihydro-1H-pyrazole-3-carboxylic acid-[1,2,4]-triazole-4-yl-amide,
• 5-(4-Chloro-phenyl)-1-(2,4-dichloro-phenyl)-4,5-dihydro-1H-pyrazole-3-carboxylic acid-(4-methyl-piperazin-1-yl)-amide,
• 5-(4-Chloro-phenyl)-1-(2,4-dichloro-phenyl)-4,5-dihydro-1H-pyrazole-3-carboxylic acid diethylamide,
• [5-(4-Chloro-phenyl)-1-(2,4-dichloro-phenyl)-4,5-dihydro-1H-pyrazole-3-yl]-piperidine-1-yl-methanone,
• N-[5-(4-Chloro-phenyl)-1-(2,4-dichlorophenyl)-4,5-dihydro-1H-pyrazole-3-carbonyl]-4-methylphenylsulfonamide,
• 5-(4-chloro-phenyl)-1-(2,4-dichlorophenyl)-4,5-dihydro-1H-pyrazol-3-carboxylic acid,
• (rac)-5-(4-ch)oro-phenyl)-1-(2,4-dich)orophenyl)-4,5-dihydro-1H-pyrazol-3-carboxylic acid,
• (R)-5-(4-chloro-phenyl)-1-(2,4-dichlorophenyl)-4,5-dihydro-1H-pyrazol-3-carboxylic acid,
• (S)-5-(4-ch)oro-phenyl)-1-(2,4-dich)orophenyl)-4,5-dihydro-1H-pyrazol-3-carboxylic acid,
or any mixture thereof
optionally in the form of a corresponding N-oxide, a corresponding salt or a corresponding solvate.

32. A medicament comprising the combination (B) according to claims 29 to 31, and optionally one or more pharmaceutically acceptable excipients.

33. Use of a combination (B) according to claims 29 to 31 for the manufacture of a medicament for the treatment of coagulation related diseases.
